(19) Europäisches Patentamt
European Patent Office
Office européen des brevets

(11) Publication number : **0 306 263 B1**

(12) # EUROPEAN PATENT SPECIFICATION

(45) Date of publication of patent specification : **18.03.92 Bulletin 92/12**

(51) Int. Cl.$^5$ : **C07D 309/30,** C07C 69/757, A61K 31/365, C07C 69/30, A61K 31/22, A61K 31/19

(21) Application number : **88308010.3**

(22) Date of filing : **30.08.88**

(54) **Novel HMG-COA reductase inhibitors.**

(30) Priority : **02.09.87 US 92353**

(43) Date of publication of application : **08.03.89 Bulletin 89/10**

(45) Publication of the grant of the patent : **18.03.92 Bulletin 92/12**

(84) Designated Contracting States : **CH DE FR GB IT LI NL**

(56) References cited :
**EP-A- 0 183 132**
**EP-A- 0 251 625**
**JP-A-59 122 483**
**US-A- 4 376 863**
**US-A- 4 448 979**
**US-A- 4 517 373**
**US-A- 4 537 859**
**US-A- 4 537 859**

(73) Proprietor : **MERCK & CO. INC.**
**126, East Lincoln Avenue P.O. Box 2000**
**Rahway New Jersey 07065-0900 (US)**

(72) Inventor : **Lee, Ta Jyh**
**1921 Supplee Road**
**Lansdale Pennsylvania 19446 (US)**
Inventor : **Stokker, Gerald E.**
**Plymouth Road**
**Gwynedd Valley Pennsylvania (US)**
Inventor : **Smith, Robert L.**
**1355 Pickwick Lane**
**Lansdale Pennsylvania 19446 (US)**

(74) Representative : **Hesketh, Alan, Dr.**
**European Patent Department Merck & Co., Inc. Terlings Park Eastwick Road**
**Harlow Essex, CM20 2QR (GB)**

**EP 0 306 263 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to new 3-hydroxy-3-methylglutaryl-coenzyme A (HMG-CoA) reductase inhibitors, to compositions containing them, and to their synthesis.

Hypercholesterolemia is known to be one of the prime risk factors for atherosclerosis and coronary heart disease, the leading cause of death and disability in western countries. To date, there is still no effective antihypercholesterolemic agent commercially available that has found wide patient acceptance. The bile acid sequestrants seem to be moderately effective but they must be consumed in large quantities, i.e., several grams at a time, and they are not very palatable.

There are agents known, however, that are very active antihypercholesterolemic agents which function by limiting cholesterol biosynthesis via inhibiting the enzyme, HMG-CoA reductase. These agents include the natural fermentation products compactin and mevinolin and a variety of semisynthetic and totally synthetic analogs thereof. The naturally occurring compounds and their semisynthetic analogs have the following general structural formulae:

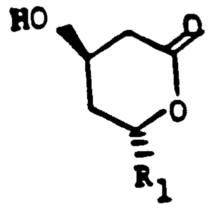

or

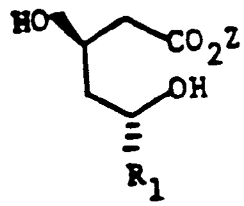

wherein:

Z is hydrogen, $C_{1-5}$ alkyl or $C_{1-5}$ alkyl substituted with a member of the group consisting of phenyl, dimethylamino, or acetylamino; and

$R_1$ is:

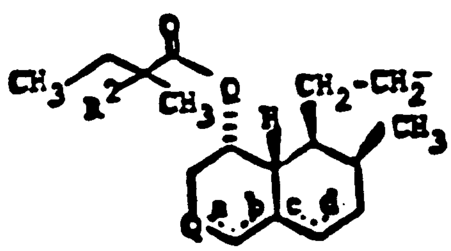

wherein Q is

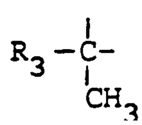

or

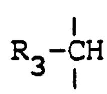

;

$R_3$ is H or OH; and

$R_2$ is hydrogen or methyl; and a, b, c, and d represent optional double bonds, especially where b and d represent double bonds or a, b, c, and d are all single bonds.

U.S. Patent 4,517,373 discloses semisynthetic hydroxy containing compounds represented by the above general formula wherein $R_1$ is

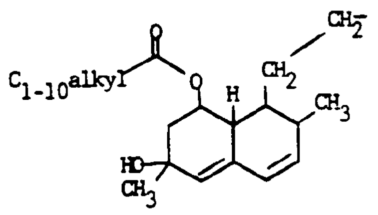

and

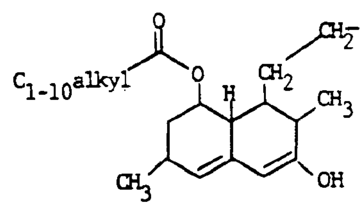

U.S. Patent 4,537,859 and U.S. Patent 4,448,979 also disclose semisynthetic hydroxy-containing compounds represented by the above general formula wherein $R_1$ is

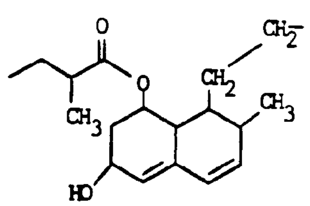

and

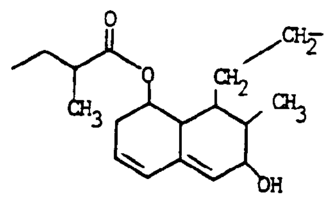

These compounds are prepared by the action of certain microorganisms on the corresponding non-hydroxylated substrates. One such organism described in U.S. 4,537,859 is of the genus Nocardia.

U.S. Patent 4,376,863 discloses a fermentation product, isolated after cultivation of a microorganism belonging to the genus Aspergillus, which has a hydroxy-containing butyryloxy side chain and is represented by the above general formula wherein $R_1$ is

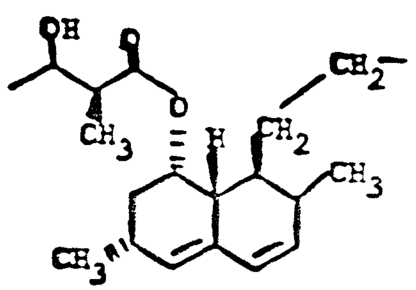

Japanese unexamined patent application J59-122,483-A discloses a semisynthetic hydroxy-containing compound represented by the above general formula wherein $R_1$ is

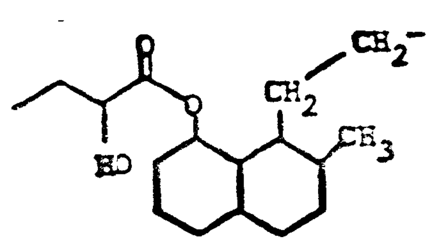

EP-A-0 251 625, a document which falls within the terms of Article 54(3) EPC, discloses compounds which are analogs of mevinolin and related compounds which possess a hydroxymethyl group, acyloxymethyl group, carbamoyloxymethyl group, a carboxy group, an alkoxycarbonyl group or a carbamoyl group substituted on the 6-position of the polyhydronaphthyl moiety.

This invention relates to novel compounds which are HGM-CoA reductase inhibitors and are useful as antihypercholesterolemic agents. Specifically, the compound of this invention are analogs of mevinolin and related compounds which possess a hydroxyalkyl group, acyloxyalkyl or carbamoyloxyalkyl group of the structure

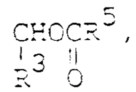

or a ketone group of the structure

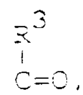

substituted on the 6-position of the polyhydronaphthyl moiety. Additionally, pharmaceutical compositions of these novel compounds, as the sole therapeutically active ingredient and in combination with bile acid sequestrants, are disclosed. Other embodiments of this invention are processes for preparing the novel compounds.

The specific HGM-CoA reductase inhibitors of this invention are the compounds represented by the following general structural formulae (I) and (II):

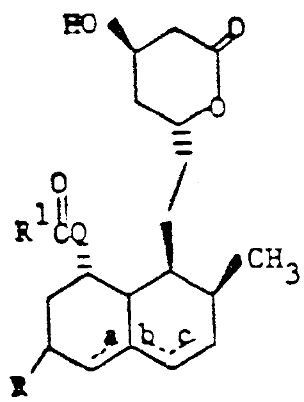

(I)

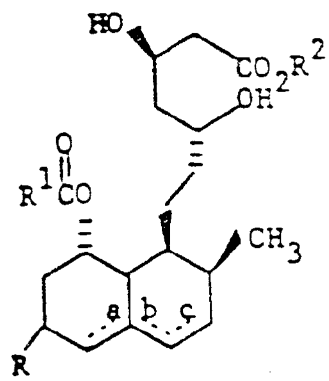

(II)

wherein:
R is

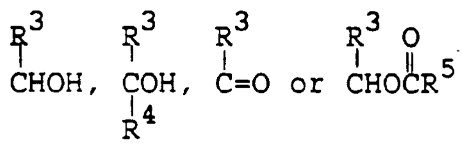

$R^1$ and $R^5$ are independently selected from:

(1) $C_{1-10}$ alkyl;
(2) substituted $C_{1-10}$ alkyl in which one or more substituent(s) is selected from
(a) halogen,
(b) hydroxy,
(c) $C_{1-10}$ alkoxy,
(d) $C_{1-5}$ alkoxycarbonyl
(e) $C_{1-5}$ acyloxy,
(f) $C_{3-8}$ cycloalkyl,
(g) phenyl,
(h) substituted phenyl in which the substituents are X and Y,
(i) $C_{1-10}$ alkylS(0)$_n$ in which n is 0 to 2,
(j) $C_{3-8}$ cycloalkylS(0)$_n$,
(k) phenylS(0)$_n$,
(l) substituted phenylS(0)$_n$ in which the substituents are X and Y, and

(m) oxo;
(3) $C_{1-10}$ alkoxy;
(4) $C_{2-10}$ alkenyl;
(5) $C_{3-8}$ cycloalkyl;
(6) substituted $C_{3-8}$ cycloalkyl in which one substituent is selected from
(a) $C_{1-10}$ alkyl
(b) substituted $C_{1-10}$ alkyl in which the substituent is selected from
(i) halogen,
(ii) hydroxy,
(iii) $C_{1-10}$ alkoxy,
(iv) $C_{1-5}$ alkoxycarbonyl,
(v) $C_{1-5}$ acyloxy,
(vi) phenyl,
(vii) substituted phenyl in which the substituents are X and Y
(viii) $C_{1-10}$ alkylS(O)$_n$,
(ix) $C_{3-8}$ cycloalkylS(O)$_n$,
(x) phenylS(0)$_n$,
(xi) substituted phenylS(O)$_n$ in which the substituents are X and Y, and
(xii) oxo,
(c) $C_{1-10}$ alkylS(0)$_n$,
(d) $C_{3-8}$ cycloalkylS(0)$_n$,
(e) phenylS(0)$_n$,
(f) substituted phenylS(0)$_n$ in which the substituents are X and Y,
(g) halogen,
(h) hydroxy,
(i) $C_{1-10}$ alkoxy,
(j) $C_{1-5}$ alkoxycarbonyl,
(k) $C_{1-5}$ acyloxy
(l) phenyl, and
(m) substituted phenyl in which the substituents are X and Y;
(7) phenyl;
(8) substituted phenyl in which the substituents are X and Y;
(9) amino;
(10) $C_{1-5}$ alkylamino;
(11) di($C_{1-5}$ alkyl)amino;
(12) phenylamino;
(13) substituted phenylamino in which the substituents are X and Y;
(14) phenyl $C_{1-10}$ alkylamino;
(15) substituted phenyl $C_{1-10}$ alkylamino in which the substituents are X and Y;
(16) a member selected from
(a) piperidinyl,
(b) pyrrolidinyl,
(c) piperazinyl,
(d) morpholinyl, and
(e) thiomorpholinyl; and
(17) $R^6$S in which $R^6$ is selected from
(a) $C_{1-10}$ alkyl,
(b) phenyl, and
(c) substituted phenyl in which the substituents are X and Y; $R^2$ is independently selected from:
(1) hydrogen;
(2) $C_{1-5}$ alkyl;
(3) substituted $C_{1-5}$ alkyl in which the substituent is selected from
(a) phenyl,
(b) dimethylamino, and
(c) acetylamino, and
(4) 2,3-dihydroxypropyl;

$R_3$ and $R_4$ are independently selected from:

(1) $C_{1-10}$ alkyl;

(2) substituted $C_{1-10}$ alkyl in which one or more substituent(s) is selected from

(a) halogen,

(b) hydroxy,

(c) $C_{1-10}$ alkoxy,

(d) $C_{1-5}$ alkoxycarbonyl,

(e) $C_{1-5}$ acyloxy,

(f) $C_{3-8}$ cycloalkyl,

(g) phenyl,

(h) substituted phenyl in which the substituents are X and Y,

(i) $C_{1-10}$ alkylS(0)$_n$,

(j) $C_{3-8}$ cycloalkylS(0)$_n$,

(k) phenylS(0)$_n$,

(l) substituted phenylS(0)$_n$ in which the substituents are X and Y, and

(m) oxo;

(3) $C_{2-10}$ alkenyl;

(4) substituted $C_{2-10}$ alkenyl in which one or more substituent(s) is selected from

(a) halogen,

(b) hydroxy,

(c) $C_{1-10}$ alkoxy,

(d) $C_{1-5}$ alkoxycarbonyl,

(e) $C_{1-5}$ acyloxy,

(f) $C_{3-8}$ cycloalkyl,

(g) phenyl,

(h) substituted phenyl in which the substituents are X and Y,

(i) $C_{1-10}$ alkylS(0)$_n$,

(j) $C_{3-8}$ cycloalkylS(0)$_n$,

(k) phenylS(0)$_n$,

(l) substituted phenylS(0)$_n$ in which the substituents are X and Y, and

(m) oxo;

(5) $C_{3-8}$ cycloalkyl;

(6) substituted $C_{3-8}$ cycloalkyl in which one substituent is selected from

(a) $C_{1-10}$ alkyl

(b) substituted $C_{1-10}$ alkyl in which the substituent is selected from

(i) halogen,

(ii) hydroxy,

(iii) $C_{1-10}$ alkoxy,

(iv) $C_{1-5}$ alkoxycarbonyl,

(v) $C_{1-5}$ acyloxy

(vi) phenyl,

(vii) substituted phenyl in which the substituents are X and Y

(viii) $C_{1-10}$ alkylS(O)$_n$,

(ix) $C_{3-8}$ cycloalkylS(O)$_n$,

(x) phenylS(O)$_n$,

(xi) substituted phenylS(O)$_n$ in which the substituents are X and Y, and

(xii) oxo,

(c) $C_{1-10}$ alkylS(0)$_n$,

(d) $C_{3-8}$ cycloalkylS(0)$_n$,

(e) phenylS(0)$_n$,

(f) substituted phenylS(0)$_n$ in which the substituents are X and Y,

(g) halogen,

(h) hydroxy,

(i) $C_{1-10}$ alkoxy,

(j) $C_{1-5}$ alkoxycarbonyl,

(k) $C_{1-5}$ acyloxy,
(l) phenyl, and
(m) substituted phenyl in which the substituents are X and Y;
(7) phenyl;
(8) substituted phenyl in which the substituents are X and Y;

X and Y independently are hydrogen, halogen, trifluoromethyl, $C_{1-3}$ alkyl, nitro, cyano or group selected from:

(1) $R^8O(CH_2)_m$ in which m is 0 to 3 and $R^8$ is hydrogen, $C_{1-3}$alkyl or hydroxy-$C_{2-3}$alkyl;

(2)

$$R^9\overset{\overset{O}{\|}}{C}O(CH_2)_m \text{ or } R^9O\overset{\overset{O}{\|}}{C}O(CH_2)_m$$

in which $R^9$ is hydrogen, $C_{1-3}$alkyl, hydroxy-$C_{2-3}$alkyl, phenyl, naphthyl, amino $C_{1-3}$alkyl, $C_{1-3}$alkylamino-$C_{1-3}$alkyl, di($C_{1-3}$alkyl)amino-$C_{1-3}$alkyl, hydroxy-$C_{2-3}$ alkylamino-$C_{1-3}$alkyl or di(hydroxy-$C_{2-3}$alkyl) amino-$C_{1-3}$alkyl;

(3)

$$R^{10}O\overset{\overset{O}{\|}}{C}(CH_2)_m$$

in which $R^{10}$ is hydrogen $C_{1-3}$alkyl, hydroxy-$C_{2-3}$ alkyl, $C_{1-3}$alkoxy-$C_{1-3}$alkyl, phenyl or naphthyl;

(4)

$$R^{11}R^{12}N(CH_2)_m,\ R^{11}R^{12}N\overset{\overset{O}{\|}}{C}(CH_2)_m$$

or

$$:\ R^{11}R^{12}N\overset{\overset{O}{\|}}{C}O(CH_2)_m$$

in which $R^{11}$ and $R^{12}$ independently are hydrogen, $C_{1-3}$ alkyl, hydroxy-$C_{2-3}$alkyl or together with the nitrogen atom to which they are attached form a heterocyclic group selected from piperidinyl, pyrrolidinyl, piperazinyl, morpholinyl or thiomorpholinyl;

(5) $R^{13}S(0)_n(CH_2)_m$ in which $R^{13}$ is hydrogen, $C_{1-3}$alkyl, amino, $C_{1-3}$alkylamino or di($C_{1-3}$alkyl)amino; and <u>a</u>, <u>b</u> and <u>c</u> each represent single bonds or one of <u>a</u>, <u>b</u> and <u>c</u> represents a double bond, or both <u>a</u> and <u>c</u> represent double bonds;

or a pharmaceutically acceptable salt thereof.

Except where specifically defined to the contrary, the terms "alkyl", "alkoxy" and "acyl" include both the straight-chain and branched-chain species of the term.

One embodiment of this invention is the class of compounds of the formulae (I) and (II) wherein:

$R^1$ and $R^5$ are independently selected from:

(1) $C_{1-10}$ alkyl;
(2) substituted $C_{1-10}$ alkyl in which one or more substituent(s) is selected from
(a) halogen,
(b) hydroxy,
(c) $C_{1-10}$ alkoxy,
(d) $C_{1-5}$ alkoxycarbonyl,

(e) $C_{1-5}$ acyloxy,
(f) $C_{3-8}$ cycloalkyl,
(g) phenyl,
(h) substituted phenyl in which the substituents are X and Y, and
(i) oxo;
(3) $C_{3-8}$ cycloalkyl;
(4) substituted $C_{3-8}$ cycloalkyl in which one substituent is selected from
(a) $C_{1-10}$ alkyl,
(b) substituted $C_{1-10}$ alkyl in which the substituent is selected from
(i) halogen,
(ii) hydroxy,
(iii) $C_{1-10}$ alkoxy
(iv) $C_{1-5}$ acyloxy,
(v) $C_{1-5}$ alkoxycarbonyl,
(vi) phenyl,
(vii) subsituted phenyl in which the substituents are X and Y, and
(viii) oxo,
(c) halogen,
(d) hydroxy,
(e) $C_{1-10}$ alkoxy,
(f) $C_{1-5}$ alkoxycarbonyl,
(g) $C_{1-5}$ acyloxy,
(h) phenyl,
(i) substituted phenyl in which the substituents are X and Y;
(5) phenylamino;
(6) substituted phenylamino in which the substituents are X and Y;
(7) phenyl $C_{1-10}$ alkylamino; and
(8) substituted phenyl $C_{1-10}$ alkylamino in which the substituents are X and Y.

One subclass of this embodiment is the compounds of the formulae (I) and (II) wherein: $R_3$ and $R_4$ are independently selected from:
(1) $C_{1-10}$ alkyl;
(2) $C_{3-8}$ cycloalkyl; and
(3) phenyl.

More specifically illustrating this subclass are the compounds wherein $R^1$ and $R^5$ are independently selected from:
(1) $C_{1-10}$ alkyl;
(2) $C_{3-8}$ cycloalkyl;
(3) phenylamino; and
(4) substituted phenylamino in which the substituents are X and Y.

Exemplifying this subclass are those compounds of the formulae (I) and(II) wherein both a and c represent double bonds, especially the following compounds:
(1) 6(R)-[2-[8(S)-(2,2-dimethylbutyryloxy)-2(S)-methyl-6(S)-(1-hydroxyethyl)-1,2,6,7,8,8a(R)-hexahydronaphthyl-1(S)]ethyl]-4(R)-hydroxy-3,4,5,6-tetrahydro-2H-pyran-2-one;
(2) 6(R)-[2-[8(S)-(2,2-dimethylbutyryloxy)-2(S)-methyl-6(R)-(1-hydroxyethyl)-1,2,6,7,7,8a(R)-hexahydronaphthyl-1(S)]ethyl]-4(R)-hydroxy-3,4,5,6-tetrahydro-2H-pyran-2-one;
(3) 6(R)-[2-[8(S)-(2-methylbutyryloxy)-2(S)-methyl-6(S)-(1-hydroxyethyl)-1,2,6,7,8,8a(R)-hexahydronaphthyl-1(S)]ethyl]-4(R)-hydroxy-3,4,5,6-tetrahydro-2H-pyran-2-one;
(4) 6(R)-[2-[8(S)-(2-methylbutyryloxy)-2(S)-methyl-6(R)-(1-hydroxyethyl)-1,2,6,7,8,8a(R)-hexahydronaphthyl-1(S)]ethyl]-4(R)-hydroxy-3,4,5,6-tetrahydro-2H-pyran-2-one; and
the corresponding ring opened dihydroxy acids, and esters thereof, particularly the methyl ester.

Also exemplifying this subclass are those compounds of the formulae (I) and(II) wherein each of a, b and c represents single bonds, especially the following compounds:
(1) 6(R)-[2-[8(S)-(2,2-dimethylbutyryloxy)-2(S)-methyl-6(S)-(1-hydroxyethyl)-1,2,3,4,4a(S),5,6,7,8,8a(S)-decahydronaphthyl-1(S)]ethyl]-4(R)-hydroxy-3,4, 5,6-tetrahydro-2H-pyran-2-one;
(2) 6(R)-[2-[8(S)-(2,2-dimethylbutyryloxy)-2(S)-methyl-6(R)-(1-hydroxyethyl)-1,2,3,4,4a(S),5,6,7,8,8a(S)-decahydronaphthyl-1(S)]ethyl]-4(R)-hydroxy-3,4,5,6-tetrahydro-2H-pyran-2-one;

(3) 6(R)-[2-[8(S)-(2,2-dimethylbutyryloxy)-2(S)-methyl-6(S)-(1-hydroxyphenylmethyl)-1,2,3,4, 4a(S),5,6,7, 8,8a(S)-decahydronaphthyl-1(S)]ethyl]-4(R)-hydroxy-3,4,5,6-tetrahydro-2H-pyran-2-one, and the corresponding ring opened dihydroxy acids, and esters, as the single isomers or a mixture thereof.

Another example of this subclass are those compounds of the formulae (I) and(II) wherein one of a, b and c represents single bonds, especially the following compounds:

(1) 6(R)-[2-(8(S)-(2,2-dimethylbutyryloxy)-2(S)-methyl-6(R)-(1-hydroxyethyl)-1,2,3,4,6,7,8,8a (R)-octahydronaphthyl-1(S)]ethyl]-4(R)-hydroxy-3,4,5,6-tetrahydro-2H-pyran-2-one; and the corresponding ring opened dihydroxy acids, and esters thereof.

The compounds of formulae (I) and (II) wherein a, b and c all represent single bonds are conveniently prepared from mevinolin via the following synthetic pathway:

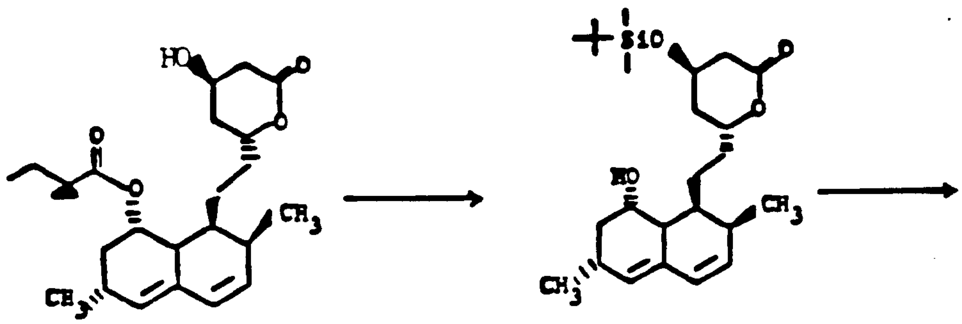

(1) (2)

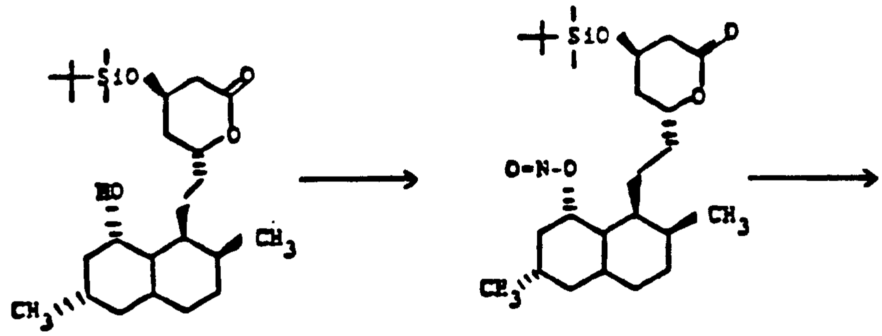

(3) (4)

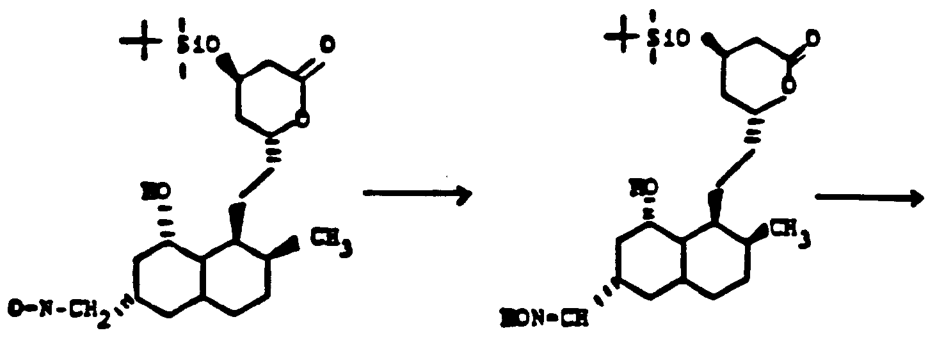

(5) (6)

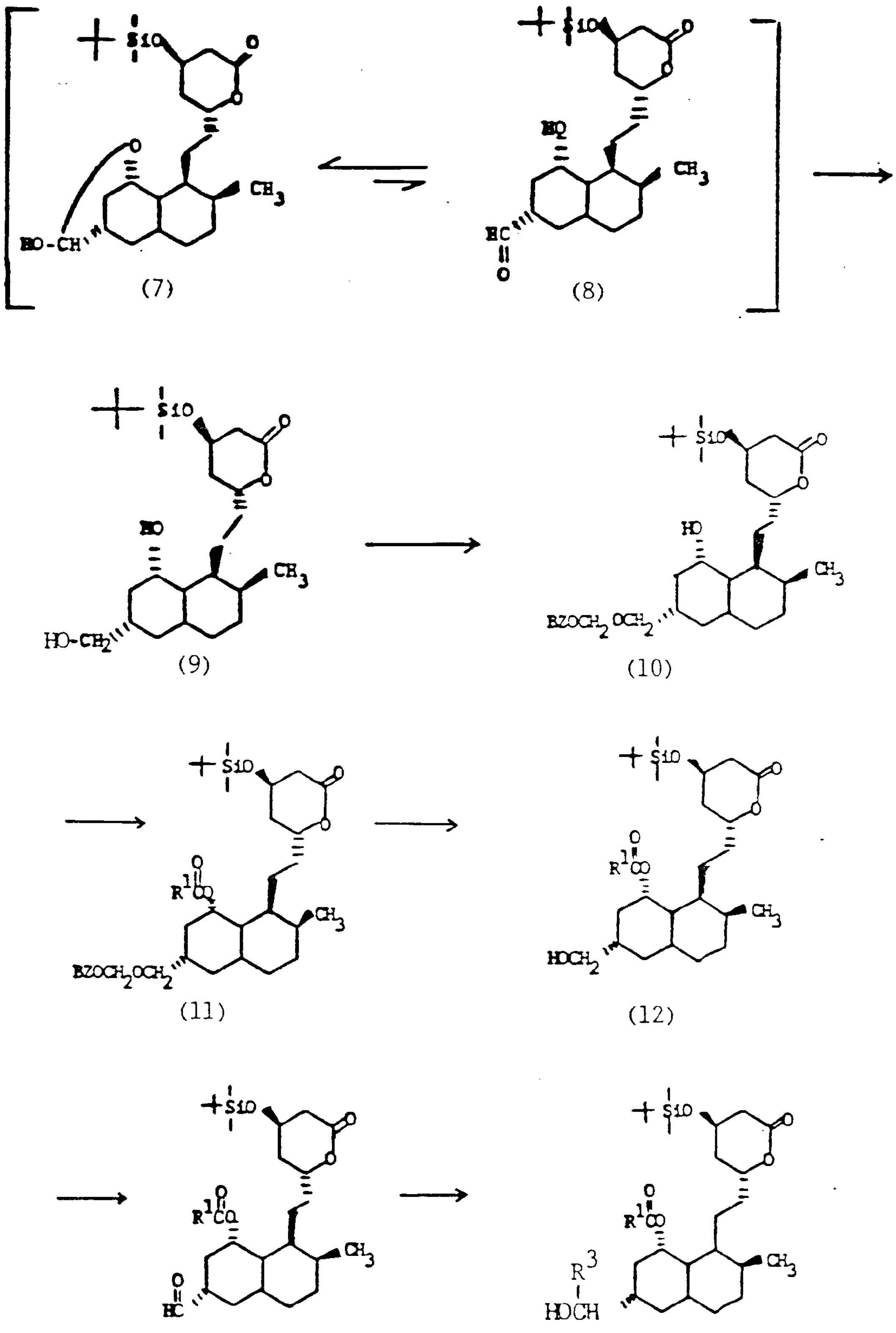

(7) (8)

(9) (10)

(11) (12)

(13) (14)

The starting material, mevinolin (1) is readily available or may be prepared according to the fermentation procedures disclosed in U.S. Patent 4,231,938. The compound of the formula (1) is hydrolyzed under the conditions disclosed in U.S. Patent 4,444,784 and then the 4-hydroxy function in the lactone moiety is protected with a suitable protecting group, exemplified here as a t-butyldimethylsilyl group, according to the procedure disclosed in U.S. Patent 4,444,784 to yield the compound (2). Compound (2) is then hydrogenated under the

analogous conditions disclosed in U.S. Patent 4,351,844 to afford the compound (3). Compound (3) is then treated with nitrosyl chloride in the presence of a base, such as a trialkylamine, specifically trimethylamine, triethylamine, pyridine, N,N-dimethylbenzylamine and the like, to afford the compound (4). Compound (4) is then subjected to a photo-rearrangement to give the compound (5). Compound (5) is heated to reflux in a protic solvent such as isopropanol and the like to yield the compound (6). Compound (6) is converted into compound (7) by treatment with an alkali metal nitrite, such as sodium nitrite or potassium nitrite, in an aqueous organic acid such as acetic acid, propionic acid or the like. Compound (7), which is a hemiacetal, is in equilibrium with the hydroxy aldehyde, compound (8). This equilibrium mixture of compound (7) and compound (8) is treated with a reducing agent, such as sodium borohydride, to afford compound (9). Compound (9), is converted to compound (10) by treatment with benzyl chloromethyl ether in the presence of a suitable base such as a trialkylamine, preferably diisopropylethylamine, in a suitable organic solvent such as methylene chloride in the cold, preferably at about 0°C. Compound (10) is acylated under suitable conditions utilizing the appropriate alkanoyl halide or alkanoic acid to afford compounds (11). Removal of the benzyloxymethyl protecting group from compounds (11) via catalytic hydrogenation under standard conditions provides compounds (12). The latter are oxidized to compounds (13) by standard methods, including the method of Swern involving the use of oxalyl chloride and dimethyl sulfoxide in methylene chloride followed by triethylamine. Compound (13) is then reacted with an appropriately substituted Grignard reagent to afford compound (14) which after removal of the trialkylsilyl ether protecting group under standard conditions yields the compounds of formula (I) wherein R is

$$\begin{array}{l}\mathrm{CHOH}\,.\\ |\\ \mathrm{R}^3\end{array}$$

The compounds of formula (I) wherein R is

$$\begin{array}{l}\mathrm{R}^3\\ |\\ \mathrm{COH}\\ |\\ \mathrm{R}^4\end{array}$$

are conveniently prepared from compound (14) by oxidizing the hydroxyalkyl group to the ketone of the structure

$$\begin{array}{l}\mathrm{R}^3\\ |\\ \mathrm{C{=}O}\,,\end{array}$$

followed by a second Grignard reaction and the removal of the trialkylsilyl ether protecting group.

The compounds of the formula (I) wherein R is

$$\begin{array}{l}\mathrm{R}^3\\ |\\ \mathrm{C{=}O}\end{array}$$

are conveniently prepared from compound (14) by oxidizing the hydroxyalkyl group to the ketone followed by the removal of the trialkylsilyl ether protecting group.

The compounds of the formulae (I) and (II) wherein a represents a double bond are conveniently prepared from the compound of the formula (2) by the hydrogenation procedure using Wilkinson's catalyst as disclosed in U.S. Patent 4,444,784 and subjecting the resultant 6(R)-[2-[8(S)-hydroxy-2(S),6(R)-dimethyl-1,2,3,4,6,7,8,8a(R)-octahydronaphthyl-1(S)]ethyl]-4(R)-(t-butyldimethylsilyloxy)-3,4,5,6-tetrahydro-2H-pyran-2-one to the above noted reaction sequence.

The compounds of the formulae (I) and (II) wherein b represents a double bond are conveniently prepared using an analogous reaction sequence but utilizing 6(R)-[2-[8(S)-hydroxy-2(S),6(S)-dimethyl-1,2,3,5,6,7,8,8a(R)-octahydronaphthyl-1(S)]ethyl]-4(R)-(t-butyldimethylsilyloxy)-3,4,5,6-tetrahydro-2H-pyran-2-one in place of the compound of the formula (3). This starting material may be prepared according to the procedures disclosed in U.S. Patent 4,444,784.

The compounds of the formulae (I) and (II) wherein c is a double bond are conveniently prepared using an

analogous reaction sequence but utilizing 6(R)-[2-[8(S)-hydroxy-2(S),6(S)-dimethyl-1,2,4a-(R),5,6,7,8,8a(S)-octahydronaphthyl-1(S)]ethyl]-4(R)-(t-butyldimethylsilyloxy)-3,4,5,6-tetrahydro-2H-pyran-2-one in place of the compound of the formula (3). This starting material may be prepared from the natural fermentation product prepared according to the procedures disclosed in U.S. Patent 4,294,846.

The compounds (13), which are prepared as illustrated in the preceeding synthetic pathway, each have the CHO group appended to the polyhydronaphthalene ring as a 6α-substituent. The corresponding 6β-epimers are conveniently prepared via the following synthetic pathway:

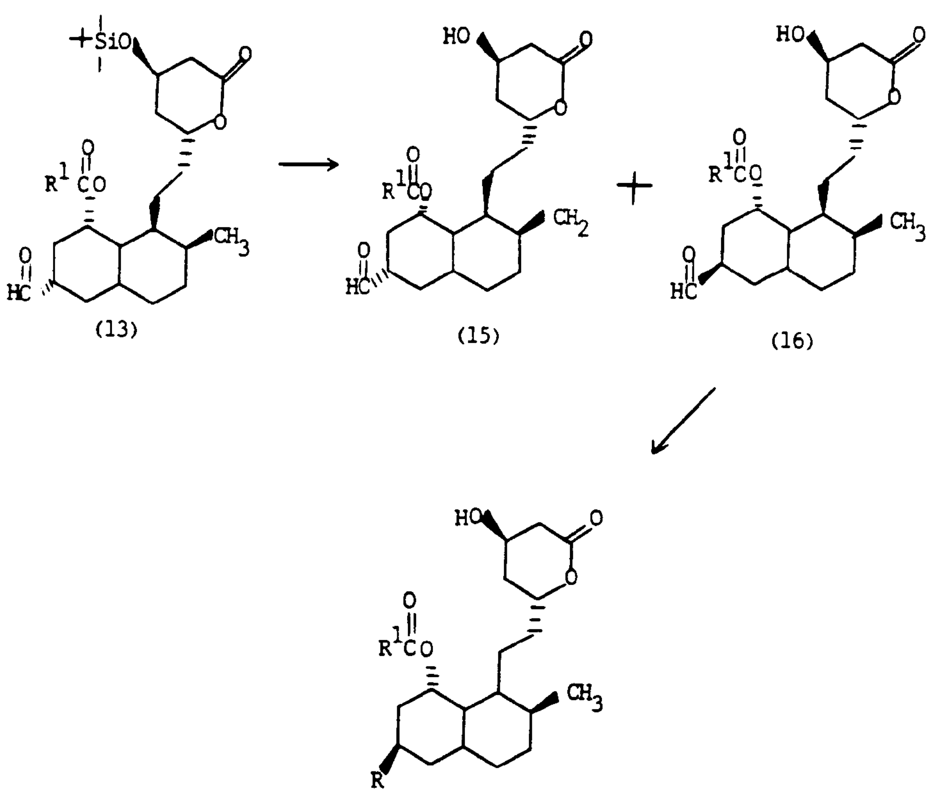

(13) (15) (16)

Treatment of compounds (13) under the conditions (i.e., tetra-n-butylammonium fluoride in THF buffered with HOAc) used to deblock the tert-butyldimethylsilyl ether serves both to deblock this protecting group in compounds (13) and to epimerize the resulting 6α-aldehydes (15) to afford a mixture of epimeric compounds (15) and (16), the ratio of the 6β-epimer to the 6α-epimer being determined by the exact reaction conditions used in each instance. After isolation by chromatography on a suitable support such as silica gel and the like, compounds (16) can be converted to compounds of this invention by utilizing analogous reaction conditions as those employed with the transformations of compound (15).

Where the product formed by the above described synthetic pathways is not the desired form of that compound, then that product may be subjected to one or more further reactions such as hydrolysis, salification, esterification, acylation, ammonolysis or lactonization by conventional methods, as described in more detail hereafter.

The starting compound may be a free carboxylic acid, its corresponding lactone or a salt (e.g., metal, amino acid or amine salt) or ester (particularly alkyl ester) thereof.

Preferred metal salts are salts with alkali metals, such as sodium or potassium, salts with alkaline earth metals, such as calcium, or salts with other metals such as magnesium, aluminum, iron, zinc, copper, nickel or cobalt, of which the alkali metal, alkaline earth metal, magnesium and aluminum salts are preferred, the sodium, calcium and aluminum salts being most preferred.

Preferred amino acids to form amino acid salts are basic amino acids, such as arginine, lysine, histidine, α,β-diaminobutyric acid or ornithine.

Preferred amines to form amine salts include t-octylamine, dibenzylamine, dichlorohexylamine, morpholine, alkyl esters of D-phenylglycine and D-glucosamine. Also preferred is ammonia to form the ammonium salt.

Esters are preferably the alkyl esters, such as the methyl, ethyl, propyl, isopropyl, butyl, isobutyl or pentyl

esters, of which the methyl ester is preferred. However, other esters such as phenyl $C_{1-5}$alkyl, dimethylamino-$C_{1-5}$alkyl, or acetylamino-$C_{1-5}$alkyl may be employed if desired.

Of the starting materials, the alkali metal salts, e.g., the sodium or potassium salts, are particularly preferred, the sodium salt being most preferred as it has been found that this gives the best conversion of the substrate into the desired product.

Where the product obtained by the processes of the present invention is a salt of the carboxylic acid of formula (II), the free carboxylic acid itself can be obtained by adjusting the pH of the filtrate to a value of 4 or less, preferably to a value of from 3 to 4. Any organic acid or mineral acid may be employed, provided that it has no adverse effect upon the desired compound. Examples of the many acids which are suitable include trifluoroacetic acid, acetic acid, hydrochloric acid and sulphuric acid. This carboxylic acid may itself be the desired product or it may be, and preferably is, subjected to subsequent reactions, as described below, optionally after such treatments as extraction, washing and lactonization.

Metal salts of the carboxylic acids of formula (II) may be obtained by contacting a hydroxide, carbonate or similar reactive compound of the chosen metal in an aqueous solvent with the carboxylic acid of formula (II). The aqueous solvent employed is preferably water, or it may be a mixture of water with an organic solvent, preferably an alcohol (such as methanol or ethanol), a ketone (such as acetone), an aliphatic hydrocarbon (such as hexane) or an ester (such as ethyl acetate). It is preferred to use a mixture of a hydrophilic organic solvent with water. Such reactions are normally conducted at ambient temperature but they may, if desired, be conducted with heating.

Amine salts of the carboxylic acids of formula (II) may be obtained by contacting an amine in an aqueous solvent with the carboxylic acid of formula (II). Suitable aqueous solvents include water and mixtures of water with alcohols (such as methanol or ethanol), ethers (such as tetrahydrofuran), nitriles (such as acetonitrile) or ketones (such as acetone); it is preferred to use aqueous acetone as the solvent for this reaction. The reaction is preferably carried out at a temperature of ambient or below, more preferably a temperature of from 5° to 10°C. The reaction immediately goes to completion. Alternatively, a metal salt of the carboxylic acid of formula (II) (which may have been obtained as described above) can be dissolved in an aqueous solvent, after which a mineral acid salt (for example the hydrochloride) of the desired amine is added, employing the same reaction conditions as when the amine itself is reacted with the carboxylic acid of formula (II) and the desired product is then obtained by a salt exchange reaction.

Amino acid salts of the carboxylic acids of formula (II) may be obtained by contacting an amino acid in aqueous solution with the carboxylic acid of formula (II). Suitable aqueous solvents include water and mixtures of water with alcohols (such as methanol or ethanol) or ethers (such as tetrahydrofuran).

Esters, preferably alkyl esters, of the carboxylic acids of formula (II) may be obtained by contacting the carboxylic acid of formula (II) with an appropriate alcohol, preferably in the presence of an acid catalyst, for example a mineral acid (such as hydrochloric acid or sulphuric acid), a Lewis acid (for example boron trifluoride) or an ion exchange resin. The solvent employed for this reaction is not critical, provided that it does not adversely affect the reaction; suitable solvents include benzene, chloroform, ethers and the like. Alternatively, the desired product may be obtained by contacting the carboxylic acid of formula (II) with a diazoalkane, in which the alkane moiety may be substituted or unsubstituted. This reaction is usually effected by contacting the acid with an ethereal solution of the diazoalkane. As a further alternative, the ester may be obtained by contacting a metal salt of the carboxylic acid of formula (II) with a halide, preferably an alkyl halide, in a suitable solvent; preferred solvents include dimethylformamide, tetrahydrofuran, dimethylsulfoxide and acetone. All of the reactions for producing esters are preferably effected at about ambient temperature, but, if required by the nature of the reaction system, the reactions may be conducted with heating.

The compounds of formula (I) in which R is

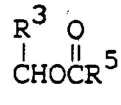

may be prepared from the compounds of formula (I) in which R is

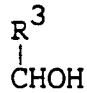

by the treatment of tert-butyldimethylsilyl chloride in the presence of an organic base to afford the compounds in which the hydroxy group of the lactone of the compounds of formula (I) are protected as a tert butyldimethylsilyl ether. Subsequent acylation using the appropriate acyl halide, anhydride, isocyanate or carbamoyl chloride, under standard conditions. Desilylation under standard conditions will afford the compounds of formula (I) in which R is

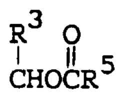.

Lactones of the carboxylic acids of formula (I) may be obtained by lactonizing the carboxylic acids of formula (II) under ordinary conditions known to one skilled in the art.

The intrinsic HMG-CoA reductase inhibition activity of the claimed compounds is measured in the in vitro protocol published in J. Med. Chem., 28, p. 347-358 (1985).

For estimation of relative inhibitory potencies, compactin was assigned a value of 100 and the $IC_{50}$ value of the test compound was compared with that of compactin determined simultaneously in the published in vitro protocol.

Representative of the intrinsic HMG-CoA reductase inhibitory activities of the claimed compounds are the relative potencies tabulated below for a number of the claimed compounds.

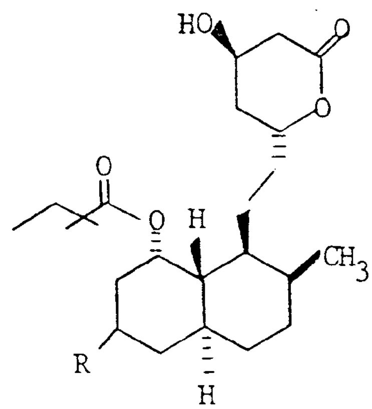

| R | Relative Potency |
|---|---|
| 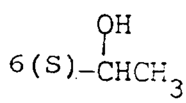 (Isomer A) | 131 |
| 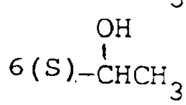 (Isomer B) | 113 |
| 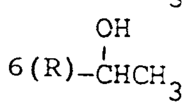 (Isomer A) | 111 |
| 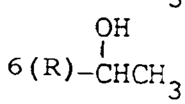 (Isomer B) | 104 |
| 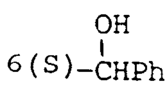 | 220 |

The compounds of this invention are useful as antihypercholesterolemic agents for the treatment of arteriosclerosis, hyperlipidemia, familial hypercholesterolemia and like diseases in humans. They may be administered orally or parenterally in the form of a capsule, a tablet, an injectable preparation or the like. It is usually desirable to use the oral route. Doses may be varied, depending on the age, severity, body weight and other conditions of human patients but daily dosage for adults is within a range of from about 2 mg to 2000 mg (preferably 10 to 100 mg) which may be given in two to four divided doses. Higher doses may be favorably employed as required.

The compounds of this invention may also be coadministered with pharmaceutically acceptable nontoxic cationic polymers capable of binding bile acids in a non-reabsorbable form in the gastro-intestinal tract. Examples of such polymers include cholestyramine, colestipol and poly[methyl-(3-trimethylaminopropyl)imino-trimethylene dihalide]. The relative amounts of the compounds of this invention and these polymers is between 1:100 and 1:15,000.

The following examples illustrate the preparation of the compounds of the formulae (I) and (II) and their incorporation into pharmaceutical compositions and as such are not to be considered as limiting the invention set forth in the claims appended hereto.

EXAMPLE 1

Preparation of 6(R)-[2-[8(S)-(2,2-dimethylbutyryloxy)-2(S)-methyl-6(S)-(1-hydroxyethyl)-1,2,3,4,4a(S),5,6,7,8,8a(S)-decahydronaphthyl-1(S)]ethyl]-4(R)-hydroxy-3,4,5,6-tetrahydro-2H-pyran-2-one

(a) 6(R)-[2-[8(S)-Nitrosyloxy-2(S),6(S)-dimethyl-1,2,3,4,4a(S),5,6,7,8,8a(S)-decahydronaphthyl-1(S)]ethyl]-4(R)-(tert-butyldimethylsilyloxy)-3,4,5,6-tetrahydro-2H-pyran-2-one (1a)

A stream of nitrosyl chloride gas was passed into a stirred solution of 6(R)-[2-[8(S)-hydroxy-2(S), 6(S)-dimethyl-1,2,3,4,4a(S),5,6,7,8,8a(S)-decahydronaphthyl-1(S)]ethyl]-4(R)-(tert-butyldimethylsilyloxy)-3,4,5,6-te trahydro-2H-pyran-2-one (800 mg, 1.82 mmol) in pyridine (14 ml) at 0°C until the solution was saturated (brownish fumes filled the reaction flask). The resulting mixture was stirred at 0°C for another 10 minutes, poured into cold water and extracted with diethyl ether. The extract was washed successively with dilute HCl, water and 5% $NaHCO_3$, dried ($MgSO_4$), filtered and concentrated in vacuo to afford the title compound as a white solid; mp 92-4°C; $^1H$ nmr ($CDCl_3$) δ 0.86 (3H, d, J=7Hz), 0.89 (9H, s), 0.99 (3H, d, J=7Hz), 2.55 (H, m of d, J=18Hz), 2.60 (H, d of d, J=18,4 Hz), 4.28 (H, m), 4.53 (H, m), 5.84 (H, m).

| | | | |
|---|---|---|---|
| Anal Calc'd for $C_{25}H_{45}NO_5Si$: | | | |
| | C, 64.20; | H, 9.70; | N, 3.00. |
| Found: | C, 64.09; | H, 10.00; | N, 3.06. |

(b) 6(R)-[2-[8(S)-Hydroxy-2(S)-methyl-6(S)-nitrosylmethyl-1,2,3,4,4a(S),5,6,7,8,8a(S)-decahydronaphthyl-1(S)]ethyl]-4(R)-(tert-butyldimethylsilyloxy)-3,4,5,6-tetrahydro-2H-pyran-2-one (1b)

Nitrogen gas was passed through a solution of compound 1a (870 mg, 1.82 mmol) in benzene (320 ml) for 25 minutes. This solution was irradiated under $N_2$ with a 450 watt Hanovia medium pressure mercury lamp (pyrex filter) for 40 minutes at room temperature. The reaction mixture was then concentrated in vacuo and the residue applied to a silica gel column. Elution of the column with methylene chloride:acetone (50:1; v:v) followed by elution with methylene chloride:acetone: isopropanol (100:10:2; v:v:v) yielded the desired product as a foamy oil; $^1H$ nmr ($CDCl_3$) δ 0.83 (3H, d, J=7Hz), 0.88 (9H, s) 4.10 (H, bs), 4.29 (H, m), 4.64 (2H, d, J=8Hz), 4.67 (H, m).

(c) 6(R)-[2-[8(S)-Hydroxy-2(S)-methyl-6(S)-hydroxyiminomethyl-1,2,3,4,4a(S),5,6,7,8,8a(S)-decahydronaphthyl-1(S)]ethyl]-4(R)-(tert-butyldimethylsilyloxy)-3,4,5,6-tetrahydro-2H-pyran-2-one (1c)

Compound 1b (288 mg, 0.616 mmol) was dissolved in isopropanol (15 ml) and heated at reflux for 2 hours. After cooling the reaction mixture was concentrated in vacuo to leave a residue which afforded the title compound as a gummy oil; $^1H$ nmr ($CDCl_3$) δ 0.86 (3H, d, J=7Hz), 0.90 (9H, s) 2.33 (H, d, i=14 Hz), 2.78 (H, m), 4.11 (H, m), 4,32 (H, m), 4.66 (H, m), 7,50 (H, d, J=6Hz).

(d) 6(R)-[2-[8-Hydroxy-2(S)-methyl-6(S)-formyl-1,2,3,4,4a(S),5,6,7,8,8a(S)-decahydronaphthyl-1(S)]-ethyl]-4(R)-(tert-butyldimethylsilyloxy)-3,4,5,6-tetrahydro-2H-pyran-2-one (1d)

Sodium nitrite (477 mg 6.83 mmol) was added at 0°C in one portion to a stirred solution of compound 1c (324 mg 0.683 mmol) in acetic acid (14 ml) and water (7 ml). The resulting mixture was stirred at 0°C for 10 minutes, warmed to room temperature and stirred for 2.5 hours. The mixture was then diluted with water and extracted with diethyl ether. This ethereal extract was washed with water, 5% $NaHCO_3$ (twice), dried and fil-

tered. Evaporation of the filtrate in vacuo afforded a brownish oily residue whose nmr spectrum is consistent with the structure for compound 1d; $^1$H nmr ($CDCl_3$) δ 0.80 (3H, d, J=7Hz), 0.88 (9H, s), 4,30 (H, m) 4.55 (2H, m).

(e) 6(R)-[2-[8(S)-Hydroxy-2(S)-methyl-6(S)-hydroxymethyl-1,2,3,4,4a(S),5,6,7,8,8a(S)-decahydronaphthyl-1(S)]ethyl]-4(R)-(tert-butyldimethylsilyloxy)-3,4,5,6-tetrahydro-2H-pyran-2-one (1e)

Powdered sodium borohydride (40 mg, 1.05 mmol) was added at 0°C to a stirred solution of compound 1d (296 mg, 0.651 mmol) in 95% ethanol (15 ml) in one portion. The resulting mixture was stirred at 0°C for 0.5 hours, then slowly treated with a solution of aqueous $(NH_4)_2SO_4$ (0.7g in 15 ml of $H_2O$). The resulting mixture was stirred at 0°C for 0.5 hours, diluted with water (60 ml) and extracted with diethyl ether. This extract was washed with water, 5% $NaHCO_3$, dried, filtered and evaporated to give a crude sample which was purified by flash chromatography. Elution of the column with methylene chloride:acetone:isopropanol (100:10:2; v:v:v) afforded the desired product as a white solid; mp 124-7°C; $^1$H nmr ($CDCl_3$) δ 0.83 (3H, d, J=7Hz), 0.90 (9H, s), 3.73 (H, d of d, J=11,6 Hz), 3.79 (H, d of d, J=11,6 Hz), 4.10 (H, bs) 4.31 (H, m), 4.70 (H, m).

| | |
|---|---|
| Anal Calc'd for $C_{25}H_{46}O_5$ Si | C, 66.03; H, 10.20. |
| Found: | C, 66.07; H, 10.38. |

(f) 6(R)-[2-[8(S)-hydroxy-2(S)-methyl-6(S)-benzyloxymethoxymethyl-1,2,3,4,4a(S),5,6,7,8,8a(S)-decahydronaphthyl-1(S)]-ethyl]-4(R)-tert-butyl-dimethylsilyloxy)-3,4,5,6-tetrahydro-2H-pyran-2-one-(1f)

To a stirred solution of compound 1e (9.7 g, 21.3 mmol) and diisopropylethylamine (10 mL, 57.4 mmol) in $CH_2Cl_2$ (25 mL) cooled to 0°C was added dropwise a solution of benzyl chloromethyl ether (3.76 g, 24 mmol) in $CH_2Cl_2$ (10 mL). The resulting mixture was stirred at 0°C for 10 minutes allowed to warm to room temperature, stirred at room temperature for 22 hours and then poured into ice water. The heterogeneous mixture was extracted with diethyl ether. The organic phase was separated, washed successively with dilute HCl, water, aqueous $NaHCO_3$ and water, dried ($Na_2SO_4$), filtered and evaporated in vacuo to afford a residue which was purified by flash chromatography on silica gel. Elution with $CH_2Cl_2$-acetone (50:1; v:v) removed the impurities. Continued elution with $CH_2Cl_2$-acetone (20:1; V:V) provided the title compound as a viscous oil; $^1$H nmr ($CDCl_3$) δ 0.82 (3H, d, J=7Hz), 0.88 (9H, s), 2.6 (2H, m), 2.70 (H, d, J=6Hz), 3.75 (2H, m), 4.0 (H, m), 4.28 (H, m), 4.60 (H, d, J=12Hz), 4.62 (H, d, J=12Hz), 4.66 (H, m), 4.78 (H, d, J=6Hz), 4.81 (H, d, J=6Hz), 7.3 (5H, m).

(g)6(R)-[2-[8(S)-(2,2-Dimethylbutyryloxy)-2(S)-methyl-6(S)-benzyloxymethoxymethyl-1,2,3,4,4a(S),5,6,7,8,8a(S)-decahydronaphthyl-1(S)]ethyl]-4(R)-(tert-butyldimethylsilyloxy)-3,4,5,6-tetrahydro-2H-pyran-2-one (1g)

Powdered lithium bromide (6.4 g, 74 mmol) was added under $N_2$ to a stirred mixture of 2,2-dimethylbutyryl chloride (4.97 g, 37 mmol) in pyridine (100 mL) and the resulting mixture was stirred and warmed to 40°C until a clear solution was obtained. To the resulting solution was added 4-dimethylaminopyridine (0.3 g, 2.45 mmol) and a solution of 1f (7.25 g, 13 mmol) in pyridine (30 mL). The resulting mixture was stirred and heated at 90°C for 3.5 hours, cooled to room temperature, poured into ice water and extracted with diethyl ether. The organic phase was separated, washed with dilute HCl, aqueous $NaHCO_3$ and saturated brine, dried ($Na_2SO_4$), filtered and evaporated in vacuo to give an oily residue which was purified by flash chromatography on silica gel. Elution with $CH_2Cl_2$-acetone (50:1; v:v) afforded the title compound as a viscous oil; $^1$H nmr ($CDCl_3$) δ 0.82 (3H, d, J=7HZ), 0.83 (3H, t, J=7Hz), 0.88 (9H, s), 1.14 (3H, s), 1.15 (3H, s), 2.67 (2H, m), 3.39 (H, d of d, J=10, 6Hz), 3.86 (H, t, J=10Hz), 4.27 (H, m), 4.54 (H, d, J=16Hz), 4.61 (H, d, J=16Hz), 4.74 (2H, s), 5.13 (H, m), 7.32 (5H, m).

(h) 6(R)-[2-[8(S)-(2,2-Dimethylbutyryloxy)-2(S)-methyl-6(S)-hydroxymethyl-1,2,3,4,4a(S),5,6,7,8,8a (S)-decahydronaphthyl-(S)]ethyl]-4(R)-(tert-butyldimethylsilyloxy)-3,4,5,6-tetrahydro-2H-pyran-2-one(1h)

A mixture of compound 1g (6.1g, 8.85 mmol), 10% Pd/C (0.5 g) and acetic acid (3 drops) in isopropanol (200 mL) was hydrogenated in a Paar apparatus for 4 hours. The resulting mixture was treated with powdered $NaHCO_3$ (1 g), stirred for 15 minutes and filtered. The filtrate was evaporated in vacuo to provide a residue which was dissolved in toluene (100 mL). The resulting solution was evaporated in vacuo to provide a residue which again was dissolved in toluene (100 mL). Evaporation of this solution in vacuo gave a residue which crystallized from diethyl ether-hexane to provide the title compound as a colorless solid, mp 70-71°C; $^1$H nmr ($CDCl_3$) δ 0.82 (3H, d, J=7Hz), 0.83 (3H, t, J=7Hz), 0.87 (9H, s), 1.15 (3H, s), 1.16 (3H, s), 2.66 (2H, m), 3.55

(H, m), 3.78 (H, m), 4.28 (H, m), 4.65 (H, m), 5.14 (H, m).

Anal. Cal'd for $C_{31}H_{56}O_6Si$: C, 67.34; H, 10.21.
Found: C, 67.21; H, 10.35.

(i) 6(R)-[2-[8(S)-(2,2-Dimethybutyryloxy)-2(S)-methyl-6(S)-formyl-1,2,3,4,4a(S),5,6,7,8,8a(S)-decahydronaphthyl-1(S)]-ethyl]-4(R)-(tert-butyldimethylsilyloxy)-3,4,5,6-tetrahydro-2H-pyran-2-one (li)

To a stirred solution of oxalyl chloride (152 mg, 1.2 mmol) in $CH_2Cl_2$ (10 ml) cooled at -78°C was added dimethylsulfoxide (156 mg, 2 mmol) via syringe under $N_2$. The resulting mixture was stirred at -78°C for 15 minutes and treated with a solution of compound 1h (383 mg, 0.693 mmol) in $CH_2Cl_2$ (5 mL) added dropwise. The resulting mixture was stirred at -78°C, for 30 minutes, treated with triethylamine (253 mg, 2.5 mmol), stirred for an additional 10 minutes at -78°C warmed to room temperature, poured into ice water and extracted with diethyl ether. The organic extract was washed with aqueous $NaHCO_3$ and water, dried ($Na_2SO_4$), filtered and evaporated in vacuo to provide the title compound as a pale yellow oil; $^1H$ nmr ($CDCl_3$) δ 0.83 (3H, d, J=7Hz), 0.83 (3H, t, J=7Hz), 0.89 (9H, s), 1.10 (3H, s), 1.12 (3H, s), 2.58 (2H, m), 4.28 (H, m), 4.55 (H, m), 5.20 (H, m), 9.63 (H, s).

(j) 6(R)-[2-[8(S)-(2,2-Dimethybutyryloxy)-2(S)-methyl-6(S)-(1-hydroxyethyl)-1,2,3,4,4a(S),5,6,7,8,8a(S)-decahydronaphthyl-1(S)]-ethyl]-4(R)-(tert-butyldimethylsilyloxy)-3,4,5,6-tetrahydro-2H-pyran-2-one(1j)

A solution of methylmagnesium bromide (3.0M in diethyl ether, 0.50 mL, 1.5 mmol) was added dropwise to a stirred solution of compound 1i (700 mg, 1.27 mmol) dissolved in dry diethyl ether (20 mL) at -78°C under an argon atmosphere. The cooling bath was removed and the reaction mixture was stirred with gradual warming to room temperature over about a 2 hour period before quenching with a saturated brine solution (10 mL). The reaction mixture was distributed between diethyl ether (100 mL) and water (50 mL). The diethyl ether layer was separated, dried, filtered and evaporated to leave crude product which was purified by flash chromatography on a silica gel column. Elution of the column with chloroform:acetone (40:1/v:v) gave the two diastereomeric alcohols as clear colorless glasses.

Isomer A: Rf=0.17, diagnostic nmr peaks ($CDCl_3$) δ 4.02 (H, m), 4.27 (H, m), 4.54 (H, m), 5.12 (H, m).

Isomer B: Rf=0.07, diagnostic nmr peaks ($CDCl_3$) δ 3.98 (H, m), 4.27 (H, m), 4.56 (H, m), 5.16 (H, m).

(k) 6(R)-[2-[8(S)-(2,2-dimethylbutyryloxy)-2(S)-methyl-6(S)-(1-hydroxyethyl)-1,2,3,4,4a,(S),5,6,7,8,8a(R)-decahydronaphthyl-1(S)]ethyl]-4(R)-hydroxy-3,4,5,6 tetrahydro-2H-pyran-2-one (Isomer A)

n-Tetrabutylammonium fluoride solution (1M in THF, 1.65 mL, 1.65 mmol) was added to a stirred solution of compound 1j isomer A (180 mg, 310 μmol) and acetic acid (180 μL) in tetrahydrofuran (3 mL). The resulting solution was stirred at room temperature for 18 hours. Thin layer chromatography now showed only a trace of silyl ether and was thus diluted with water (30 mL) and the crude product was extracted into diethyl ether (100 mL). The etherial extract was washed with 0.1N hydrochloric acid (100 mL), water (2 X 50 mL), saturated sodium bicarbonate solution (50 mL), dried, filtered, and evaporated to yield the crude alcohol which was purified by flash chromatography on silica gel. Elution of the column with methylene chloride:acetone (6:1/v:v) gave the desired diol as a clear colorless glass: Rf=0.46 chloroform:acetone (4:l/v:v), diagnostic nmr peaks ($CDCl_3$) δ 2.6 (H, md, J=18Hz), 2.73 (H, dd, J=5, 18Hz), 4.03 (H, m), 4.35 (H,m), 4.56 (H,m), 5.15 (H,m).

Anal Calc'd for $C_{26}H_{44}O_6$
C, 68.99; H, 9.80.
Found C, 69.07; H, 9.82.

(1) 6(R)-[2-[8(S)-(2,2-dimethylbutyryloxy)-2(S)-methyl-6(S)-(1-hydroxyethyl)-1,2,3,4,4a,(S),5,6,7,8,8a(R)-decahydronaphthyl-1(S)]ethyl]-4(R)-hydroxy-3,4,5,6-tetrahydro-2H-pyran-2-one (Isomer B)

Aqueous hydrofluoric acid (49%, 0.42 mL) was added dropwise to a stirred solution of compound 1j isomer B (200 mg, 0.34 mmol) in acetonitrile (8 mL) at about 10°C. The cooling bath was removed and the clear colorless solution was stirred at room temperature for 1 1/4 hour. The reaction mixture was distributed between diethyl ether (100 mL) and saturated sodium bicarbonate solution (50 mL). The ethereal layer was dried, filtered, and evaporated to provide crude diol which was purified by flash chromatography on silica gel. Elution of the

column with methylene chloride:acetone (6:1/v:v) gave the desired diol as a clear colorless glass:$R_f$=0.31 chloroform:acetone (4:1/v:v), diagnostic nmr peaks ($CDCl_3$) δ 2.6 (H, md, J=18Hz), 2.73 (H, dd, J=5,18Hz), 3.98 (H, m), 4.14 (H, m), 4.56 (H,m), 5.18 (H, m). MS (FAB)m/z 453 ($MH^+$).

| | |
|---|---|
| Anal Calc'd for | $C_{26}H_{44}O_6$ |
| | C, 68.99; H, 9.80. |
| Found | C, 68.96; H, 10.04. |

EXAMPLE 2

(j) 6(R)-[2-[8(S)-(2,2-Dimethylbutyryloxy)-2(S)-methyl-6(S)-(α-hydroxybenzyl)-1,2,3,4,4a(S),5,6,7,8,8a(S)-decahydronaphthyl-1(S)]ethyl]-4(R)-(tert-butyldimethylsilyloxy)-3,4,5,6-tetrahydro-2H-pyran-2-one (2j)

Utilizing the general method described in Example 1 step j except that the methylmagnesium bromide was replaced by phenylmagnesium chloride the above titled compound was obtained as a single diastereomer.

(k) 6(R)-[2-[8(S)-(2,2-Dimethylbutyryloxy)-2(S)-methyl-(S)-(α-hydroxybenzyl)-1,2,3,4,4a(S),5,6,7,8,8a(S)-decahydronaphthyl-1(S)]ethyl]-4(R)-hydroxy-3,4,5,6-tetrahydro-2H-pyran-2-one (2k)

Utilizing the general method described in Example 1 step k except that 1j isomer A was replaced by 2j, the above titled compound was obtained as a colorless solid following recrystallization from acetonitrile-water, mp 196-196.5°C, diagnostic nmr peaks ($CDCl_3$) δ 2.64 (H, md, J=18Hz), 2.74 (H, dd, J=5, 18Hz), 4.35 (H, m) 4.59 (H, m), 4.88 (H, dd, J=3, 10.8 Hz), 5.29 (H, d, J=3Hz).

| | |
|---|---|
| Anal. Calc'd for | $C_{31}H_{46}O_6$ |
| | C, 72.34; H, 9.01 |
| Found: | C, 72.29; H, 9.29 |

EXAMPLE 3

Preparation of 6(R)-[2-[8(S)-(2,2-dimethylbutyryloxy-2(S)-methyl-6(S)-(1-oxoethyl)-1,2,3,4,4a(S),5,6,7,8,8a(S)-decahydronaphthyl-1(S)]ethyl]-4(R)-hydroxy-3,4,5,6-tetrahydro-2H-pyran-2-one

(a) 6(R)-[2-[8(S)-(2,2-dimethylbutyryloxy)-2(S)-methyl-6(S)-(1-oxoethyl)-1,2,3,4,4a(S),5,6,7,8,8a(S)-decahydronaphthyl-1(S)]ethyl]-4(R)-(tert-butyldimethylsilyloxy)-3,4,5,6-tetrahydro-2H-pyran-2-one (3a)

Pyridinium chlorochromate supported on alumina ($PCC/Al_2O_3$)(2.6 g, ca. 2.6 mmol) was added in one portion to a solution of 1j isomers A and B (575 mg, 1.0 mmol) in toluene (12 mL). The orange slurry was stirred vigorously at room temperature for 20 hours before being quenched with 2-propanol (2mL). After stirring for a further 1/2 hour the mixture was evaporated under reduced pressure at <30°C. The residue was suspended in chloroform and purified by flash chromatography on a silica gel column. Elution of the column with chloroform:acetone (100:1/v:v) gave the ketone (3a) as a colorless gum, diagnostic nmr peaks ($CDCl_3$) δ 2.10 (3H, S), 4.27 (H, m), 4.54 (H, m), 5.12 (H, m).

(b) 6(R)-[2-[8(S)-(2,2-dimethylbutyryloxy)-2(S)-methyl-6(S)-(1-oxoethyl)-1,2,3,4,4a(S),5,6,7,8,8a(S)-decahydronaphthyl-1(S)]ethyl]-4(R)-hydroxy-3,4,5,6-tetrahydro-2H-pyran-2-one (3b)

Utilizing the general method described in Example 1 step k except that 1j isomer A was replaced by 3a, the above titled compound was obtained as colorless crystals, mp 147.5-148°C after recrystallization from n-butylchloride:hexane (1:4/v:v). Diagnostic peaks in the nmr ($CDCl_3$) δ 2.10 (3H,s), 2.53 (H,m), 2.61 (H, md, J=18Hz), 2.73 (H,dd,J=5, 18Hz), 4.36 (H,m), 4.56 (H,m), 5.16 (H,m).

EXAMPLE 4

Preparation of 6(R)-[2-[8(S)-(2,2-dimethylbutyryloxy)-2(S)-methyl-6(R)-(1-hydroxyethyl)-1,2,3,4,4a(S),5,6,7,8,8a(S)decahydronaphthyl-1(S)]ethyl]-4(R)-hydroxy-3,4,5,6-tetrahydro-2H-pyran-2-one

A solution of 3b (230 mg, 0.51 mmol) in ethanol (5 mL) was treated with aqueous sodium hydroxide (0.1N, 11 mL, 1.1 mmol) and water (50 mL) and then the clear solution was stirred at 40°C for 4 days. The clear colorless solution was cooled to room temperature and sodium borohydride (50 mg, 1.2 mmol) was added. After stirring at room temperature for 1 hour chloroform (100 ml) was added and the pH of the reaction mixture was lowered to ca 1-2 with 1N HCl. The organic layer was separated and the aqueous layer was extracted with fresh chloroform (2x50mL). The organic layers were combined and dried ($MgSO_4$) before being treated with solid 1-ethyl-3-(3-dimethylaminopropyl)carbodiimide hydrochloride (200 mg., 1.04 mmol) under an atmosphere of argon.
After stirring at room temperature for 1 hour the chloroform solution was washed with water (3x100 mL), dried, filtered, and evaporated to leave crude product which was purified by flash chromatography on a silica gel column. Elution of the column with chloroform:acetone (9:1/v:v) for the first 650 mL followed by a 7:1/v:v mixture for the next 600 mL and finally a 5:1/v:v mixture gave the two diastereomeric alcohols as clear colorless glasses.

Isomer A: $R_f$ = 0.31 at 3:1/v:v, diagnostic nmr peaks ($CDCl_3$) δ 3.50 (H, m), 4.16 (H, m), 4.58 (H, m), 5.22 (H, m).

| | |
|---|---|
| Anal. Calc'd for $C_{26}H_{44}O_6 \bullet 0.15\underline{M}CH_2Cl_2$ | |
| | C, 67,49; H, 9.60 |
| Found | C, 67.31; H, 9.68 |

Isomer B: $R_f$ = 0.25 at 3:1/v:v, diagnostic nmr peaks ($CDCl_3$) δ 3.60 (H, m), 4.36 (H, m), 4.58 (H, m), 5.24 (H, m).
Anal. Found C, 67.37; H, 9.81

EXAMPLE 5

Preparation of Ammonium Salts of Compounds II

The lactone 2k (1.0 mmol) from Example 2 is dissolved with stirring in 0.1N NaOH (1.1 mmol) at ambient temperature. The resulting solution is cooled and acidified by the dropwise addition of 1N HCl. The resulting mixture is extracted with diethyl ether and the extract washed with brine and dried ($MgSO_4$). The $MgSO_4$ is removed by filtration and the filtrate saturated with ammonia (gas) to give a gum which solidified to provide the ammonium salt.

EXAMPLE 6

Preparation of Alkali and Alkaline Earth Salts of Compounds II

To a solution of 42 mg of lactone 2k from Example 2 in 2 ml of ethanol is added 1 ml of aqueous NaOH (1 equivalent). After one hour at room temperature, the mixture is taken to dryness *in vacuo* to yield the desired sodium salt.

In like manner, the potassium salt is prepared using one equivalent of potassium hydroxide, and the calcium salt, using one equivalent of CaO.

EXAMPLE 7

Preparation of Ethylenediamine Salts of Compounds II

To a solution of 0.50 g of the ammonium salt from Example 5 in 10 ml of methanol is added 75 ml of ethylenediamine. The methanol is stripped off under vacuum to obtain the desired ethylenediamine salt.

EXAMPLE 8

Preparation of Tris(hydroxymethyl)aminomethane Salts of Compounds II

To a solution of 202 mg of the ammonium salt from Example 5 in 5 ml of methanol is added a solution of 60.5 mg of tris(hydroxymethyl) aminomethane in 5 ml of methanol. The solvent is removed in vacuo to afford the desired tris(hydroxymethyl)aminomethane salt.

EXAMPLE 9

Preparation of L-Sysine Salts of Compounds II

A solution of 0.001 mole of L-lysine and 0.0011 mole of the ammonium salt from Example 5 in 15 ml of 85% ethanol is concentrated to dryness in vacuo to give the desired L-lysine salt.

Similarly prepared are the L-arginine, L-ornithine, and N-methylglucamine salts.

EXAMPLE 10

Preparation of Tetramethylammonium Salts of Compounds II

A mixture of 68 mg of ammonium salt from Example 5 in 2 ml of methylene chloride and 0.08 ml of 24% tetramethylammonium hydroxide in methanol is diluted with ether to yield the desired tetramethylammonium salt.

EXAMPLE 11

Preparation of Methyl Esters of Compounds II

To a solution of 400 mg of lactone 2k from Example 2 in 100 ml of absolute methanol is added 10 ml 0.1 M sodium methoxide in absolute methanol. This solution is allowed to stand at room temperature for one hour, then is diluted with water and extracted twice with ethyl acetate. The organic phase is separated, dried ($Na_2SO_4$), filtered and evaporated in vacuo to yield the desired methyl ester.

In like manner, by the use of equivalent amounts of propanol, butanol, isobutanol, t-butanol, amylalcohol, isoamylalcohol, 2-dimethylaminoethanol, benzylalcohol, phenethanol, 2-acetamidoethanol and the like, the corresponding esters are obtained.

EXAMPLE 12

Preparation of Free Dihydroxy Acids

The sodium salt of the compound II from Example 6 is dissolved in 2 ml of ethanol-water (1:1; v:v) and added to 10 ml of 1N hydrochloric acid from which the dihydroxy acid is extracted with ethyl acetate. The organic extract is washed once with water, dried ($Na_2SO_4$), and evaporated in vacuo with a bath temperature not exceeding 30°C. The dihydroxy acid derivative derived slowly reverts to the corresponding, parent lactone on standing.

EXAMPLE 13

As a specific embodiment of a composition of this invention, 20 mg of lactone 2k from Example 2, is formulated with sufficient finely divided lactose to provide a total amount of 580 to 590 mg to fill a size 0, hard-gelatin capsule.

## Claims

1. A compound represented by the following general structural formulae (I) and (II):

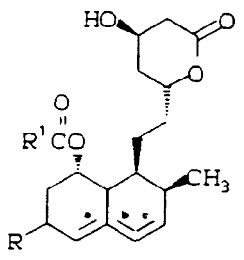

(I)

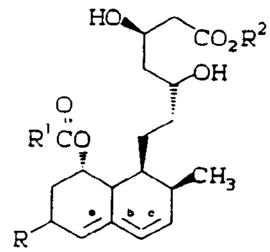

(II)

wherein:

R is

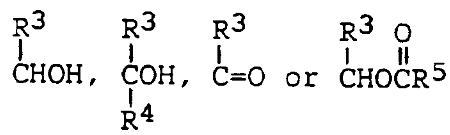

$R^1$ and $R^5$ are independently selected from:

(1) $C_{1-10}$ alkyl;

(2) substituted $C_{1-10}$ alkyl in which one or more substituent(s) is selected from

(a) halogen,

(b) hydroxy,

(c) $C_{1-10}$ alkoxy,

(d) $C_{1-5}$ alkoxycarbonyl,

(e) $C_{1-5}$ acyloxy,

(f) $C_{3-8}$ cycloalkyl,

(g) phenyl,

(h) substituted phenyl in which the substituents are X and Y,

(i) $C_{1-10}$ alkylS(0)$_n$ in which n is 0 to 2,

(j) $C_{3-8}$ cycloalkylS(0)$_n$,

(k) phenylS(0)$_n$,

(l) substituted phenylS(0)$_n$ in which the substituents are X and Y, and

(m) oxo;

(3) $C_{1-10}$ alkoxy;

(4) $C_{2-10}$ alkenyl;

(5) $C_{3-8}$ cycloalkyl;

(6) substituted $C_{3-8}$ cycloalkyl in which one substituent is selected from

(a) $C_{1-10}$ alkyl

(b) substituted $C_{1-10}$ alkyl in which the substituent is selected from

(i) halogen,

(ii) hydroxy,

(iii) $C_{1-10}$ alkoxy,

(iv) $C_{1-5}$ alkoxycarbonyl,

(v) $C_{1-5}$ acyloxy,

(vi) phenyl,

(vii) substituted phenyl in which the substituents are X and Y

(viii) $C_{1-10}$ alkylS(0)$_n$,

(ix) $C_{3-8}$ cycloalkylS(0)$_n$,

(x) phenylS(0)$_n$,

(xi) substituted phenylS(0)$_n$ in which the substituents are X and Y, and

(xii) oxo,

(c) $C_{1-10}$ alkylS(0)$_n$,

(d) $C_{3-8}$ cycloalkylS(0)$_n$,
(e) phenylS(0)$_n$,
(f) substituted phenylS(0)$_n$ in which the substituents are X and Y,
(g) halogen,
(h) hydroxy,
(i) $C_{1-10}$ alkoxy,
(j) $C_{1-5}$ alkoxycarbonyl,
(k) $C_{1-5}$ acyloxy,
(l) phenyl, and
(m) substituted phenyl in which the substituents are X and Y;
(7) phenyl;
(8) substituted phenyl in which the substituents are X and Y;
(9) amino;
(10) $C_{1-5}$ alkylamino;
(11) di($C_{1-5}$ alkyl)amino;
(12) phenylamino;
(13) substituted phenylamino in which the substituents are X and Y;
(14) phenyl $C_{1-10}$ alkylamino;
(15) substituted phenyl $C_{1-10}$ alkylamino in which the substituents are X and Y;
(16) a member selected from
(a) piperidinyl,
(b) pyrrolidinyl,
(c) piperazinyl,
(d) morpholinyl, and
(e) thiomorpholinyl; and
(17) $R^6S$ in which $R^6$ is selected from
(a) $C_{1-10}$ alkyl,
(b) phenyl, and
(c) substituted phenyl in which the substituents are X and Y; $R_2$ is independently selected from:
(1) hydrogen;
(2) $C_{1-5}$ alkyl;
(3) substituted $C_{1-5}$ alkyl in which the substituent is selected from
(a) phenyl,
(b) dimethylamino, and
(c) acetylamino, and
(4) 2,3-dihydroxypropyl;
$R_3$ and $R_4$ are independently selected from:
(1) $C_{1-10}$ alkyl;
(2) substituted $C_{1-10}$ alkyl in which one or more substituent(s) is selected from
(a) halogen,
(b) hydroxy,
(c) $C_{1-10}$ alkoxy,
(d) $C_{1-5}$ alkoxycarbonyl
(e) $C_{1-5}$ acyloxy,
(f) $C_{3-8}$ cycloalkyl,
(g) phenyl,
(h) substituted phenyl in which the substituents are X and Y,
(i) $C_{1-10}$ alkylS(0)$_n$,
(j) $C_{3-8}$ cycloalkylS(0)$_n$,
(k) phenylS(0)$_n$,
(l) substituted phenylS(0)$_n$ in which the substituents are X and Y, and
(m) oxo;
(3) $C_{2-10}$ alkenyl;
(4) substituted $C_{2-10}$ alkenyl in which one or more substituent(s) is selected from
(a) halogen,
(b) hydroxy,

(c) $C_{1-10}$ alkoxy,
(d) $C_{1-5}$ alkoxycarbonyl,
(e) $C_{1-5}$ acyloxy,
(f) $C_{3-8}$ cycloalkyl,
(g) phenyl,
(h) substituted phenyl in which the substituents are X and Y,
(i) $C_{1-10}$ alkylS(0)$_n$ ,
(j) $C_{3-8}$ cycloalkylS(0)$_n$,
(k) phenylS(0)$_n$,
(l) substituted phenylS(0)$_n$ in which the substituents are X and Y, and
(m) oxo;

(5) $C_{3-8}$ cycloalkyl;
(6) substituted $C_{3-8}$ cycloalkyl in which one substituent is selected from

(a) $C_{1-10}$ alkyl
(b) substituted $C_{1-10}$ alkyl in which the substituent is selected from

(i) halogen,
(ii) hydroxy,
(iii) $C_{1-10}$ alkoxy,
(iv) $C_{1-5}$ alkoxycarbonyl,
(v) $C_{1-5}$ acyloxy
(vi) phenyl,
(vii) substituted phenyl in which the substituents are X and Y
(viii) $C_{1-10}$ alkylS(0)$_n$,
(ix) $C_{3-8}$ cycloalkylS(0)$_n$,
(x) phenylS(0)$_n$,
(xi) substituted phenylS(0)$_n$ in which the substituents are X and Y, and
(xii) oxo,

(c) $C_{1-10}$ alkylS(0)$_n$,
(d) $C_{3-8}$ cycloalkylS(0)$_n$,
(e) phenylS(0)$_n$,
(f) substituted phenylS(0)$_n$ in which the substituents are X and Y,
(g) halogen,
(h) hydroxy,
(i) $C_{1-10}$ alkoxy,
(j) $C_{1-5}$ alkoxycarbonyl,
(k) $C_{1-5}$ acyloxy,
(l) phenyl, and
(m) substituted phenyl in which the substituents are X and Y;

(7) phenyl;
(8) substituted phenyl in which the substituents are X and Y;

X and Y independently are hydrogen, halogen, trifluoromethyl, $C_{1-3}$ alkyl, nitro, cyano or group selected from:

(1) $R^8O(CH_2)_m$ in which m is 0 to 3 and $R^8$ is hydrogen, $C_{1-3}$alkyl or hydroxy-$C_{2-3}$alkyl;
(2)

$$R^9\overset{\overset{O}{\|}}{C}O(CH_2)_m \text{ or } R^9O\overset{\overset{O}{\|}}{C}O(CH_2)_m$$

in which $R^9$ is hydrogen, $C_{1-3}$alkyl, hydroxy-$C_{2-3}$alkyl, phenyl, naphthyl, amino-$C_{1-3}$alkyl, $C_{1-3}$alkylamino-$C_{1-3}$alkyl, di($C_{1-3}$alkyl)amino-$C_{1-3}$alkyl, hydroxy-$C_{2-3}$ alkylamino-$C_{1-3}$alkyl or di(hydroxy-$C_{2-3}$alkyl) amino-$C_{1-3}$alkyl;
(3)

$$R^{10}O\overset{O}{\overset{\|}{C}}(CH_2)_m$$

in which $R^{10}$ is hydrogen, $C_{1-3}$alkyl, hydroxy-$C_{2-3}$ alkyl, $C_{1-3}$alkoxy-$C_{1-3}$alkyl, phenyl or naphthyl,

(4) $R^{11}R^{12}N(CH_2)_m$

$$R^{11}R^{12}N\overset{O}{\overset{\|}{C}}(CH_2)_m$$

or

$$R^{11}R^{12}N\overset{O}{\overset{\|}{C}}O(CH_2)_m$$

in which $R^{11}$ and $R^{12}$ independently are hydrogen, $C_{1-3}$ alkyl, hydroxy $C_{2-3}$alkyl or together with the nitrogen atom to which they are attached form a heterocyclic group selected from piperidinyl, pyrrolidinyl, piperazinyl, morpholinyl or thiomorpholinyl;

(5) $R^{13}S(O)_n(CH_2)_m$ in which $R^{13}$ is hydrogen, $C_{1-3}$alkyl, amino, $C_{1-3}$alkylamino or di($C_{1-3}$alkyl)amino; and

a, b and c each represent single bonds or one of a, b and c represents a double bond or both a and c represent double bonds;

or a pharmaceutically acceptable salt thereof.

2. A compound of Claim 1 wherein:

$R^1$ and $R^5$ are independently selected from:

(1) $C_{1-10}$ alkyl;

(2) substituted $C_{1-10}$ alkyl in which one or more substituent(s) is selected from

(a) halogen,

(b) hydroxy,

(c) $C_{1-10}$ alkoxy,

(d) $C_{1-5}$ alkoxycarbonyl,

(e) $C_{1-5}$ acyloxy,

(f) $C_{3-8}$ cycloalkyl,

(g) phenyl,

(h) substituted phenyl in which the substituents are X and Y, and

(i) oxo;

(3) $C_{3-3}$ cycloalkyl;

(4) substituted $C_{3-8}$ cycloalkyl in which one substituent is selected from

(a) $C_{1-10}$ alkyl,

(b) substituted $C_{1-10}$ alkyl in which the substituent is selected from

(i) halogen,

(ii) hydroxy

(iii) $C_{1-10}$ alkoxy

(iv) $C_{1-5}$ acyloxy,

(v) $C_{1-5}$ alkoxycarbonyl,

(vi) phenyl,

(vii) substituted phenyl in which the substituents are X and Y, and

(viii) oxo,

(c) halogen,

(d) hydroxy,

(e) $C_{1-10}$ alkoxy,

(f) $C_{1-5}$ alkoxycarbonyl,

(g) $C_{1-5}$ acyloxy,

(h) phenyl,

(i) substituted phenyl in which the substituents are X and Y;

(5) phenylamino;
(6) substituted phenylamino in which the substituents are X and Y;
(7) phenyl $C_{1-10}$ alkylamino; and
(8) substituted phenyl $C_{1-10}$ alkylamino in which the substituents are X and Y.

3. A compound of Claim 2 wherein:
$R_1$ and $R_5$ are independently selected from:
(1) $C_{1-10}$ alkyl;
(2) $C_{3-8}$ cycloalkyl;
(3) phenylamino; and
(4) substituted phenylamino in which the substituents are X and Y; and
$R_3$ and $R_4$ are independently selected from:
(1) $C_{1-10}$ alkyl;
(2) $C_{3-8}$ cycloalkyl; and
(3) phenyl.

4. A compound of Claim 3 wherein a and c represent double bonds.

5. A compound of Claim 4 which is selected from:
(1) 6(R)-[2-[8(S)-(2,2-dimethylbutyryloxy)-2(S)-methyl-6(S)-(1-hydroxyethyl)-1,2,6,7,8,8a (R)-hexahydronaphthyl-1(S)]ethyl]-4(R)-hydroxy-3,4,5,6-tetrahydro-2H-pyran-2-one;
(2) 6(R)-[2-[8(S)-(2,2-dimethylbutyryloxy)-2(S)-methyl-6(R)-(1-hydroxyethyl)-1,2,6,7,8,8a-(R)-hexahydronaphthyl-1(S)]ethyl]-4(R)-hydroxy-3,4,5,-tetrahydro-2H-pyran-2-one; and
(3) 6(R)-[2-[8(S)-(2-methylbutyryloxy)-2(S)-methyl-6(S)-(1-hydroxyethyl)-1,2,6,7,8,8a(R)-hexahydronaphthyl-1(S)]ethyl]-4(R)-hydroxy-3,4,5,6-tetrahydro-2H-pyran-2-one;
(4) 6(R)-[2-[8(S)-(2-methylbutyryloxy)-2(S)-methyl-6(R)-(1-hydroxyethyl)-1,2,6,7,8,8a(R)-hexahydronaphthyl-1(S)]ethyl]-4(R)-hydroxy-3,4,5,6-tetrahydro-2H-pyran-2-one, and
the corresponding ring opened dihydroxy acids, and esters thereof.

6. A compound of Claim 3 wherein a, b and c represent single bonds or one of a, b or c represents a double bond.

7. A compound of Claim 6 which is selected from:
(1) 6(R)-[2-[8(S)-(2,2-dimethylbutyryloxy)-2(S)-methyl-6(S)-(1-hydroxyethyl)-1,2,3,4,4a( S),5,6,7,8,8a(S)-decahydro-naphthyl-1(S)]ethyl]-4(R)-hydroxy-3,4,5,6 tetrahydro-2H-pyran-2-one;
(2) 6(R)-[2-[8(S)-(2,2-dimethylbutyryloxy)-2(S)-methyl-6(R)-(1 hydroxyethyl)-1,2,3,4,4a(S),5,6,7,8,8a(S)-decahydro-naphthyl-1(S)]ethyl]-4(R)-hydroxy-3,4,5,6-tetrahydro-2H-pyran-2-one;
(3) 6(R)-[2-[8(S)-(2,2-dimethylbutyryloxy)-2(S)-methyl-6(S)-(1-hydroxyphenylmethyl)-1,2,3,4, 4a(S),5,6,7,8,8a(S)-decahydronaphthyl-1(S)]ethyl]-4(R)-hydroxy-3,4,5,6-tetrahydro-2H-pyran-2-one;
(4) 6(R)-[2-[8(S)-(2,2-dimethylbutyryloxy)-2(S)-methyl-6(S)-(1-oxoethyl)-1,2,3,4,4a( S),5,6,7,8,8a(S)-decahydronaphthyl-1(S)]ethyl]-4(R)-hydroxy-3,4,5,6-tetrahydro-2H-pyran-2-one;
(5) 6(R)-[2-(8(S)-(2,2-dimethylbutyryloxy)-2(S)-methyl-6(R)-(1-hydroxyethyl)-1 2,3,4,6,7,8,8a(R)-octahydronaphthyl-1(S)]ethyl]-4(R)-hydroxy-3,4,5,6-tetrahydro-2H-pyran-2-one; and
the corresponding ring opened dihydroxy acids and esters thereof.

8. A hypocholesterolemic, hypolipidemic pharmaceutical composition comprising a pharmaceutically acceptable carrier and a nontoxic effective amount of a compound as defined in Claim 1.

9. A process for the preparation of a compound represented by the following general structural formula (I) according to Claim 1 which comprises:
(A) treating a compound represented by the following structural formula:

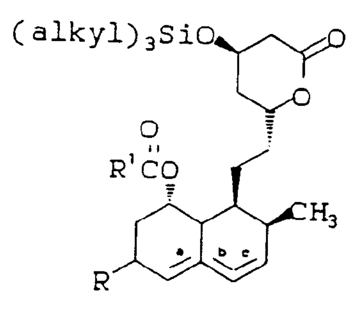

with either n-tetrabutylammonium fluoride in acetic acid and an inert solvent or aqueous hydrofluoric acid in an inert solvent; and

(B) optionally forming the compound represented by the general structural formula (II) or a pharmaceutically acceptable salt thereof by treating the compound represented by the general structural formula (I) with:

(1) an alkali metal hydroxide in an aqueous solvent to prepare the alkali metal salt; or

(2) an alkali metal hydroxide in an aqueous solvent to prepare the alkali metal salt and acidifying the salt to prepare the free dihydroxy acid; or

(3) an alkali metal hydroxide in an aqueous solvent to prepare the alkali metal salt and esterifying the salt with a $C_{1-5}$ aklanol to prepare the $C_{1-5}$ alkyl ester.

## Patentansprüche

1. Verbindung, wiedergegeben durch die nachstehenden allgemeinen Strukturformeln (I) und (II):

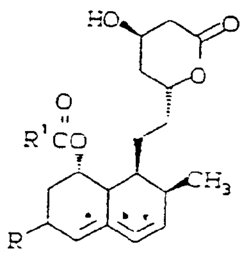
(I)

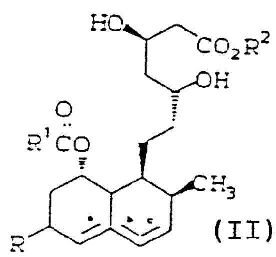
(II)

worin R

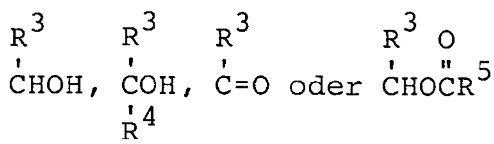

ist;

$R^1$ und $R^5$ unabhängig ausgewählt sind aus:

(1) $C_{1-10}$-Alkyl;

(2) substituiertem $C_{1-10}$-Alkyl, worin ein oder mehrere Substituenten ausgewählt sind aus

(a) Halogen,

(b) Hydroxy,

(c) $C_{1-10}$-Alkoxy,

(d) $C_{1-5}$-Alkoxycarbonyl,

(e) $C_{1-5}$-Acyloxy,

(f) $C_{3-8}$-Cycloalkyl,

(g) Phenyl,

(h) substituiertem Phenyl, worin die Substituenten X und Y sind,

(i) $C_{1-10}$-AlkylS(O)$_n$, worin n 0 bis 2 ist,

(j) $C_{3-8}$-CycloalkylS(O)$_n$,

(k) PhenylS(O)$_n$,

(l) substituiertem PhenylS(O)$_n$, worin die Substituenten X und Y sind, und

(m) Oxo;

(3) $C_{1-10}$-Alkoxy;

(4) $C_{2-10}$-Alkenyl;

(5) $C_{3-8}$-Cycloalkyl;

(6) substituiertem $C_{3-8}$-Cycloalkyl, worin ein Substituent ausgewählt ist aus

(a) $C_{1-10}$-Alkyl,

(b) substituiertem $C_{1-10}$-Alkyl, worin der Substituent ausgewählt ist aus

(i) Halogen,

(ii) Hydroxy

(iii) $C_{1-10}$-Alkoxy,
(iv) $C_{1-5}$-Alkoxycarbonyl,
(v) $C_{1-5}$-Acyloxy,
(vi) Phenyl,
(vii) substituiertem Phenyl, worin die Substituenten X und Y sind,
(viii) $C_{1-10}$-Alkyl$(O)_n$,
(ix) $C_{3-8}$-CycloalkylS$(O)_n$,
(x) PhenylS$(O)_n$,
(xi) substituiertem PhenylS$(O)_n$, worin die Substituentem X und Y sind und
(xii) Oxo,
(c) $C_{1-10}$-AlkylS$(O)_n$,
(d) $C_{3-8}$-CycloalkylS$(O)_n$,
(e) Phenyl$(O)_n$,
(f) substituiertem PhenylS$(O)_n$ worin die Substituenten X und Y sind,
(g) Halogen,
(h) Hydroxy,
(i) $C_{1-10}$-Alkoxy,
(j) $C_{1-5}$Alkoxyxcarbonyl,
(k) $C_{1-5}$-Acyloxy,
(l) Phenyl und
(m) substituiertem Phenyl, worin die Substituenten X und Y sind;
(7) Phenyl;
(8) substituiertem Phenyl, worin sie Substituenten X und Y sind;
(9) Amino;
(10) $C_{1-5}$-Alkylamino;
(11) Di($C_{1-5}$-alkyl)amino;
(12) Phenylamino;
(13) substituiertem Phenylamino, worin die Substituenten X und Y sind;
(14) Phenyl-$C_{1-10}$-alkylamino;
(15) substituiertem Phenyl-$C_{1-10}$-alkylamino, worin die Substituenten X und Y sind;
(16) ein Glied, ausgewählt aus
(a) Piperidinyl,
(b) Pyrrolidinyl,
(c) Piperazinyl,
(d) Morpholinyl und
(e) Thiomorpholinyl und
(17) $R^6$S, worin $R^6$ aus,ewählt ist aus
(a) $C_{1-10}$-Alkyl,
(b) Phenyl und
(c) substituiertem Phenyl, worin die Substituenten X und Y sind; $R^2$ unabhängig ausgewählt ist aus:
(1) Wasserstoff;
(2) $C_{1-5}$-Alkyl;
(3) substituiertem $C_{1-5}$-Alkyl, worin der Substituent ausgewählt ist aus
(a) Phenyl,
(b) Dimethylamino und
(c) Acetylamino und
(4) 2,3-Dihydroxypropyl; $R^3$ und $R^4$ unabhängig ausgewählt sind aus:
(1) $C_{1-10}$-Alkyl;
(2) substituiertem $C_{1-10}$-Alkyl, worin ein oder mehrere Substituenten ausgewählt sind aus
(a) Halogen,
(b) Hydroxy,
(c) $C_{1-10}$-Alkoxy,
(d) $C_{1-5}$-Alkoxycarbonyl,
(e) $C_{1-5}$-Acyloxy,
(f) $C_{3-8}$-Cycloalkyl,
(g) Phenyl,

(h) substituiertem Phenyl, worin die Substituenten X und Y sind,
(i) $C_{1-10}$-AlkylS(O)$_n$,
(j) $C_{3-8}$-CycloalkylS(O)$_n$,
(k) PhenylS(O)$_n$,
(l) substituiertem PhenylS(O)$_n$, worin die Substituenten X und Y sind und
(m) Oxo;
(3) $C_{2-10}$-Alkenyl;
(4) substituiertem $C_{2-10}$-Alkenyl, worin ein oder mehrere Substituenten ausgewählt sind aus
(a) Halogen,
(b) Hydroxy,
(c) $C_{1-10}$-Alkoxy,
(d) $C_{1-5}$-Alkoxycarbonyl,
(e) $C_{1-5}$-Acyloxy,
(f) $C_{3-8}$-Cycloalkyl,
(g) Phenyl,
(h) substituiertem Phenyl, worin die Substituenten X und Y sind,
(i) $C_{1-10}$-AlkylS(O)$_n$,
(j) $C_{3-8}$-CycloalkylS(O)$_n$,
(k) PhenylS(O)$_n$,
(l) substituiertem PhenylS(O)$_n$, worin die Substituenten X und Y sind, und
(m) Oxo;
(5) $C_{3-8}$-Cycloalkyl;
(6) substituiertem $C_{3-8}$-Cycloalkyl, worin ein Substituent ausgewählt ist aus
(a) $C_{1-10}$-Alkyl,
(b) substituiertem $C_{1-10}$-Alkyl, worin der Substituent ausgewählt ist aus
(i) Halogen,
(ii) Hydroxy,
(iii) $C_{1-10}$-Alkoxy,
(iv) $C_{1-5}$-Alkoxycarbonyl,
(v) $C_{1-5}$-Acyloxy,
(vi) Phenyl,
(vii) substituiertem Phenyl, worin die Substituenten X und Y sind,
(viii) $C_{1-10}$-AlkylS(O)$_n$,
(ix) $C_{3-8}$-CycloalkylS(O)$_n$,
(x) PhenylS(O)$_n$,
(xi) substituiertem PhenylS(O)$_n$, worin die Substituenten X und Y sind, und
(xii) Oxo,
(c) $C_{1-10}$-AlkylS(O)$_n$,
(d) $C_{3-8}$-CycloalkylS(O)$_n$,
(e) PhenylS(O)$_n$,
(f) substituiertem PhenylS(O)$_n$, worin die Substituenten X und Y sind,
(g) Halogen,
(h) Hydroxy,
(i) $C_{1-10}$-Alkoxy,
(j) $C_{1-5}$-Alkoxycarbonyl,
(k) $C_{1-5}$-Acyloxy,
(l) Phenyl und
(m) substituiertem Phenyl, worin die Substituenten X und Y sind;
(7) Phenyl;
(8) substituiertem Phenyl, worin die Substituenten X und Y sind;

X und Y unabhängig Wasserstoff, Halogen, Trifluormethyl, $C_{1-3}$-Alkyl, Nitro, Cyan oder eine Gruppe sind, ausgewählt aus:

(1) $R^8O(CH_2)_m$, worin m 0 bis 3 ist und $R^8$ Wasserstoff, $C_{1-3}$-Alkyl oder Hydroxy-$C_{2\ 3}$-alkyl ist;
(2)

$$R^9\overset{O}{\overset{\|}{C}}O(CH_2)_m \text{ oder } R^9O\overset{O}{\overset{\|}{C}}O(CH_2)_m,$$

worin $R^9$ Wasserstoff, $C_{1-3}$-Alkyl, Hydroxy-$C_{2-3}$-alkyl, Phenyl, Naphthyl, Amino-$C_{1-3}$-alkyl, $C_{1-3}$-Alkylamino-$C_{1-3}$-alkyl, Di($C_{1-3}$-alkyl)amino-$C_{1-3}$-alkyl, Hydroxy-$C_{2-3}$-alkylamino-$C_{1-3}$-alkyl oder Di(hydroxy-$C_{2-3}$-alkyl)amino-$C_{1-3}$-alkyl ist;

(3)

$$R^{10}O\overset{O}{\overset{\|}{C}}(CH_2)_m,$$

worin $R^{10}$ Wasserstoff, $C_{1-3}$-Alkyl, Hydroxy-$C_{2-3}$-alkyl, $C_{1-3}$-Alkoxy-$C_{1-3}$-alkyl, Phenyl oder Naphthyl ist;

(4) $R^{11}R^{12}(CH_2)_m$,

$$R^{11}R^{12}N\overset{O}{\overset{\|}{C}}(CH_2)_m \text{oder } R^{11}R^{12}N\overset{O}{\overset{\|}{C}}O(CH_2)_m,$$

worin $R^{11}$ und $R^{12}$ unabhängig Wasserstoff, $C_{1-3}$-Alkyl, Hydroxy-$C_{2-3}$-alkyl sind oder zusammen mit dem Stickstoffatom, an das sie gebunden sind, eine aus Piperidinyl, Pyrrolidinyl, Piperazinyl, Morpholinyl oder Thiomorpholinyl ausgewählte heterocyclische Gruppe bilden;

(5) $R^{13}S(O)_n(CH_2)_m$, worin $R^{13}$ Wasserstoff, $C_{1-3}$-Alkyl, Amino, $C_{1-3}$-Alkylamino oder Di($C_{1-3}$-alkyl)amino ist und

a, b und c jeweils Einzelbindungen darstellen oder eines aus a, b und c eine Doppelbindung darstellt oder sowohl a als auch c Doppelbindungen darstellen;
oder ein pharmazeutisch annehmbares Salz davon.

2. Verbindung nach Anspruch 1, worin
$R^1$ und $R^5$ unabhängig ausgewählt sind aus:

(1) $C_{1-10}$-Alkyl;
(2) substituiertem $C_{1-10}$-Alkyl, worin ein oder mehrere Substituenten ausgewählt sind aus
  (a) Halogen,
  (b) Hydroxy,
  (c) $C_{1-10}$-Alkoxy,
  (d) $C_{1-5}$-Alkoxycarbonyl,
  (e) $C_{1-5}$-Acyloxy,
  (f) $C_{3-8}$-Cycloalkyl,
  (g) Phenyl,
  (h) substituiertem Phenyl, worin die Substituenten X und Y sind und
  (i) Oxo;
(3) $C_{3-3}$-Cycloalkyl;
(4) substituiertem $C_{3-8}$-Cycloalkyl, worin ein Substituent ausgewählt ist aus
  (a) $C_{1-10}$-Alkyl,
  (b) substituiertem $C_{1-10}$-Alkyl, worin der Substituent ausgewählt ist aus
    (i) Halogen,
    (ii) Hydroxy,
    (iii) $C_{1-10}$-Alkoxy,
    (iv) $C_{1-5}$-Acyloxy,
    (v) $C_{1-5}$-Alkoxycarbonyl,
    (vi) Phenyl,
    (vii) substituiertem Phenyl, worin die Substituenten X und Y sind, und
    (viii) Oxo,
  (c) Halogen,
  (d) Hydroxy,
  (e) $C_{1-10}$-Alkoxy,

(f) $C_{1-5}$-Alkoxycarbonyl,
(g) $C_{1-5}$-Acyloxy,
(h) Phenyl,
(i) substituiertem Phenyl, worin die Substituenten X und Y sind;
(5) Phenylamino;
(6) substituiertem Phenylamino, worin die Substituenten X und Y sind;
(7) Phenyl-$C_{1-10}$-alkylamino und
(8) substituiertem Phenyl-$C_{1-10}$-alkylamino, worin die Substituenten X und Y sind.

3. Verbindung nach Anspruch 2, worin
$R^1$ und $R^5$ unabhängig ausgewählt sind aus:
(1) $C_{1-10}$-Alkyl;
(2) $C_{3-8}$-Cycloalkyl;
(3) Phenylamino und
(4) substituiertem Phenylamino, worin die Substituenten X und Y sind, und
$R^3$ und $R^4$ unabhängig ausgewählt sind aus:
(1) $C_{1-10}$-Alkyl;
(2) $C_{3-8}$-Cycloalkyl und
(3) Phenyl.

4. Verbindung nach Anspruch 3, worin a und c Doppelbindungen darstellen.

5. Verbindung nach Anspruch 4, welche ausgewählt ist aus:
(1) 6(R)-[2-[8(S)-(2,2-Dimethylbutyryloxy)-2(S)-methyl-6(S)-(1-hydroxyethyl)-1,2,6,7,8,8a(R)-hexahydronaphthyl-1(S)]ethyl]-4(R)-hydroxy-3,4,5,6-tetrahydro-2H-pyran-2-on;
(2) 6(R)-[2-[8(S)-(2,2-Dimethylbutyryloxy)-2(S)-methyl-6(R)-(1-hydroxyethyl)-1,2,6,7,8,8a(R)-hexahydronaphthyl-1(S)]ethyl]-4(R)-hydroxy-3,4,5,6-tetrahydro-2H-pyran-2-on und
(3) 6(R)-[2-[8(S)-(2-Methylbutyryloxy)-2(S)-methyl-6(S)-(1-hydroxyethyl)-1,2,6,7,8,8a(R)-hexahydronaphthyl-1(S)]ethyl]-4(R)-hydroxy-3,4,5,6-tetrahydro-2H-pyran-2-on;
(4) 6(R)-[2-[8(S)-(2-Methylbutyryloxy)-2(S)-methyl-6(R)-(1-hydroxyethyl)-1,2,6,7,8,8a(R)-hexahydronaphthyl-1(S)] ethyl]-4(R)-hydroxy-3,4,5,6-tetrahydro-2H-pyran-2-on sowie
die entsprechenden ringgeöffneten Hydroxysäuren und Ester davon.

6. Verbindung nach Anspruch 3, worin a, b und c Einfachbindungen darstellen oder eines aus a, b oder c eine Doppelbindung darstellt.

7. Verbindung nach Anspruch 6, welche ausgewählt ist aus:
(1) 6(R)-[2-[8-(S)-(2,2-Dimethylbutyryloxy)-2(S)-methyl-6(S)-(1-hydroxyethyl)-1,2,3,4,4a(S),5,6,7,8,8a(S)-decahydronaphthyl-1(S)]ethyl]-4(R)-hydroxy-3,4,5,6-tetrahydro-2H-pyran-2-on;
(2) 6(R)-[2-[8-(S)-(2,2-Dimethylbutyryloxy)-2(S)-methyl-6(R)-(1-hydroxyethyl)-1,2,3,4,4a(S),5,6,7,8,8a (S)-decahydronaphthyl-1(S)]ethyl]-4(R)-hydroxy-3,4,5,6-tetrahydro-2H-pyran-2-on;
(3) 6(R)-[2-[8(S)-(2,2-Dimethylbutyryloxy)-2(S)-methyl-6(S)-(1-hydroxyphenylmethyl)-1,2,3,4,4a(S),5,6,7,8,8a-(S)-decahydronaphthyl-1(S)]ethyl]-4(R)-hydroxy-3,4,5,6-tetrahydro-2H-pyran-2-on;
(4) 6(R)-[2-[8(S)-(2,2-Dimethylbutyryloxy)-2(S)-methyl-6(S)-(1-oxoethyl)-1,2,3,4,4a(S),5,6,7,8,8a(S)-decahydronaphthyl-1(S)]ethyl]-4(R)-hydroxy-3,4,5,6-tetrahydro-2H-pyran-2-on;
(5) 6(R)-[2-[8(S)-(2,2-Dimethylbutyryloxy)-2S)-methyl-6(R)-(1-hydroxyethyl)-1,2,3,4,6,7,8,8a(R)-octahydronaphthyl-1(S)]ethyl]-4(R)-hydroxy-3,4,5,6-tetrahydro-2H-pyran-2-on; sowie
die entsprechenden ringgeöffneten Dihydroxysäuren und Ester davon.

8. Hypocholesterolämische, hypolipidämische, pharmazeutische Zusammensetzung, welche einen pharmazeutisch annehmbaren Träger und eine nicht toxische wirksame Menge einer Verbindung nach Anspruch 1 enthält.

9. Verfahren zur Herstellung einer von der folgenden Strukturformel (I) nach Anspruch 1 wiedergegebenen Verbindung durch
(A) Behandeln einer durch die nachstehende Strukturformel wiedergegebenen Verbindung:

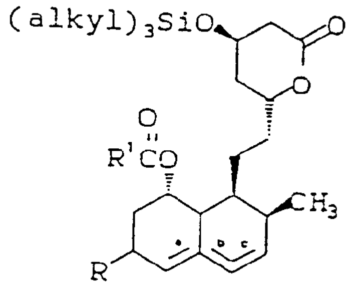

mit entweder n-Tetrabutylammoniumfluorid in Essigsäure und einem inerten Lösungsmittel oder wässriger Fluorwasserstoffsäure in einem inerten Lösungsmittel; und

(B) gegebenenfalls Bilden der durch die all gemeine Strukturformel (II) wiedergegebenen Verbindung oder eines pharmazeutisch annehmbaren Salzes davon durch Behandeln der durch die allgemeine Strukturformel (I) wiedergegebenen Verbindung mit

(1) einem Alkalimetallhydroxid in einem wässrigen Lösungsmittel zur Herstellung des Alkalimetallsalzes; oder

(2) einem Alkalimetallhydroxid in einem wässrigen Lösungsmittel zur Herstellung des Alkalimetallsalzes und Ansäuern des Salzes zur Herstellung der freien Hydroxysäure oder

(3) einem Alkalimetallhydroxid in einem wässrigen Lösungsmittel zur Herstellung des Alkalimetallsalzes und Verestern des Salzes mit einem $C_{1-5}$-Alkanol zur Herstellung des $C_{1-5}$-Alkylesters.

## Revendications

1. Composé représenté par les formules générales structurales I et II :

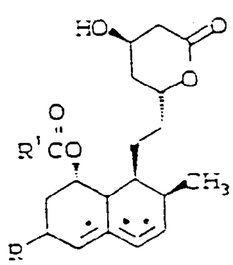

(I)

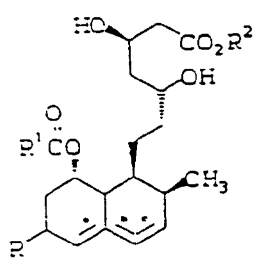

(II)

dans lesquelles:
R représente

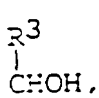, 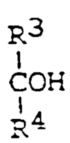, 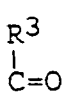 ou 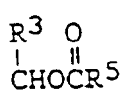

$R^1$ et $R^5$ sont indépendamment choisis parmi :

(1) alkyle en $C_{1-10}$ ;

(2) alkyle en $C_{1-10}$ substitué dont un ou plusieurs substituant(s) est (sont) choisi(s) parmi :

(a) halogène,

(b) hydroxy,

(c) alcoxy en $C_{1-10}$,

(d) alcoxycarbonyle en $C_{1-5}$,

(e) acyloxy en $C_{1-5}$,

(f) cycloalkyle en $C_{3-8}$,
(g) phényle,
(h) phényle substitué dont les substituants sont X et Y,
(i) alkyle en $C_{1-10}$ $S(0)_n$ dans lequel n est 0 à 2,
(j) cycloalkyle en $C_{3-8}S(0)_n$,
(k) phényle $S(0)_n$,
(l) phényle substitué $S(0)_n$ dans lequel les substituants sont X et Y, et
(m) oxo ;
(3) alcoxy en $C_{1-10}$ ;
(4) alcényle en $C_{2-10}$ ;
(5) cycloalkyle en $C_{3-8}$ ;
(6) cycloalkyle en $C_{3-8}$ substitué dans lequel un substituant est choisi parmi :
(a) alkyle en $C_{1-10}$,
(b) alkyle substitué en $C_{1-10}$ dans lequel le substituant est choisi parmi :
(i) halogène,
(ii) hydroxy,
(iii) alcoxy en $C_{1-10}$,
(iv) alcoxycarbonyle en $C_{1-5}$,
(v) acyloxy en $C_{1-5}$,
(vi) phényle,
(vii) phényle substitué dans lequel les substituants sont X et Y,
(viii) alkyle en $C_{1-10}$ $S(0)_n$,
(ix) cycloalkyle en $C_{3-8}$ $S(0)_n$,
(x) phényle $S(0)_n$,
(xi) phényle $S(0)_n$ substitué dont les substituants sont X et Y, et
(xii) oxo ;
(c) alkyle en $C_{1-10}$ $S(0)_n$,
(d) cycloalkyle en $C_{3-8}$ $S(0)_n$,
(e) phényle $S(0)_n$,
(f) phényle $S(0)_n$ substitué dont les substituants sont X et Y,
(g) halogène,
(h) hydroxy,
(i) alcoxy en $C_{1-10}$,
(j) alcoxycarbonyle en $C_{1-5}$,
(k) acyloxy en $C_{1-5}$,
(l) phényle, et
(m) phényle substitué dont les substituants sont X et Y ;
(7) phényle ;
(8) phényle substitué dont les substituants sont X et Y ;
(9) amino ;
(10) alkylamino en $C_{1-5}$ ;
(11) dialkylamino en $C_{1-5}$ ;
(12) phénylamino ;
(13) phénylamino substitué dont les substituants sont X et Y ;
(14) phénylalkylamino en $C_{1-10}$ ;
(15) phénylalkylamino en $C_{1-10}$ substitué dont les substituants sont X et Y ;
(16) un élément choisi parmi :
(a) pipéridinyle,
(b) pyrrolidinyle,
(c) pipérazinyle,
(d) morpholinyle, et
(e) thiomorpholinyle ; et
(17) $R^6S$ dans lequel $R^6$ est choisi parmi :
(a) alkyle en $C_{1-10}$,
(b) phényle, et
(c) phényle substitué dont les substituants sont X et Y ; $R^2$ est indépendamment choisi parmi :

(1) hydrogène,
(2) alkyle en $C_{1-5}$,
(3) alkyle substitué en $C_{1-5}$ dont le substituant est choisi parmi :
(a) phényle,
(b) diméthylamino, et
(c) acétylamino, et
(4) 2,3-dihydroxypropyle ; $R^3$ et $R^4$ sont indépendamment choisis parmi :
(1) alkyle en $C_{1-10}$;
(2) alkyle en $C_{1-10}$ substitué dont un ou plusieurs substituant(s) est (sont) choisi(s) parmi :
(a) halogène,
(b) hydroxy,
(c) alcoxy en $C_{1-10}$ ;
(d) alcoxycarbonyle en $C_{1-5}$,
(e) acyloxy en $C_{1-5}$,
(f) cycloalkyle en $C_{3-8}$,
(g) phényle,
(h) phényle substitué dont les substituants sont X et Y,
(i) alkyle en $C_{1-10}$ $S(0)_n$,
(j) cycloalkyle en $C_{3-8}$ $S(0)_n$,
(k) phényle $S(0)_n$,
(l) phényle substitué $S(0)_n$ dans lequel les substituants sont X et Y , et
(m) oxo ;
(3) alcényle en $C_{2-10}$ ;
(4) alcényle en $C_{2-10}$ substitué dont un ou plusieurs substituant(s) est (sont) choisi(s) parmi :
(a) halogène,
(b) hydroxy,
(c) alcoxy en $C_{1-10}$,
(d) alcoxycarbonyle en $C_{1-5}$,
(e) acyloxy en $C_{1-5}$,
(f) cycloalkyle en $C_{3-8}$,
(g) phényle,
(h) phényle substitué dont les substituants sont X et Y,
(i) alkyle en $C_{1-10}$ $S(0)_n$,
(j) cycloakyle en $C_{3-8}$ $S(0)_n$,
(k) phényle $S(0)_n$,
(l) phényle $S(0)_n$ substitué dans lequel les substituants sont X et Y, et
(m) oxo ;
(5) cycloalkyle en $C_{3-8}$ ;
(6) cycloalkyle en $C_{3-8}$ substitué dans lequel un substituant est choisi parmi :
(a) alkyle en $C_{1-10}$,
(b) alkyle en $C_{1-10}$ substitué dans lequel le substituant est choisi parmi :
(i) halogène,
(ii) hydroxy,
(iii) alcoxy en $C_{1-10}$,
(iv) alcoxycarbonyle en $C_{1-5}$,
(v) acyloxy en $C_{1-5}$,
(vi) phényle,
(vii) phényle substitué dont les substituants sont X et Y,
(viii) alkyle en $C_{1-10}$ $S(0)_n$,
(ix) cycloalkyle en $C_{3-8}$ $S(0)_n$,
(x) phényle $S(O)_n$,
(xi) phényle substitué $S(0)_n$ dont les substituants sont X et Y , et
(xii) oxo,
(c) alkyle en $C_{1-10}$ $S(0)_n$,
(d) cycloalkyle en $C_{3-8}$ $S(0)_n$,

(e) phényle $S(0)_n$,
(f) phényle $S(0)_n$ substitué dont les substituants sont X et Y,
(g) halogène,
(h) hydroxy,
(i) alcoxy en $C_{1-10}$,
(j) alcoxycarbonyle en $C_{1-5}$,
(k) acyloxy en $C_{1-5}$,
(l) phényle, et
(m) phényle substitué dont les substituants sont X et Y ;

(7) phényle,
(8) phényle substitué dont les substituants sont X et Y ; X et Y représentent indépendamment un atome d'hydrogène, d'halogène, le trifluorométhyle, un radical alkyle en $C_{1-3}$, nitro, cyano ou un groupe choisi parmi :
(1) $R^80(CH_2)_m$ dans lequel m est 0 à 3 et $R^8$ est un atome d'hydrogène, un radical alkyle en $C_{1-3}$ ou hydroxyalkyle en $C_{2-3}$,
(2)

$$R^9\overset{O}{\overset{\|}{C}}O(CH_2)_m \quad ou \quad R^9O\overset{O}{\overset{\|}{C}}O(CH_2)_m$$

dans lequel $R^9$ est un atome d'hydrogène, un radical alkyle en $C_{1-3}$, hydroxyalkyle en $C_{2-3}$, phényle, napthyle, aminoalkyle en $C_{1-3}$, alkyl($C_{1-3}$) aminoalkyle en $C_{1-3}$ di(alkyl en $C_{1-3}$)aminoalkyle en $C_{1-3}$, hydroxyalkyl($C_{2-3}$)aminoalkyle en $C_{1-3}$ ou di(hydroxyalkyl $C_{2-3}$)aminoalkyle en $C_{1-3}$ ;
(3)

$$R^{10}O\overset{O}{\overset{\|}{C}}(CH_2)_m$$

dans lequel $R^{10}$ représente un atome d'hydrogène, un radical alkyle en $C_{1-3}$, hydroxyalkyle en $C_{2-3}$, alcoxy($C_{1-3}$)alkyle en $C_{1-3}$, phényle ou naphtyle ;
(4) $R^{11}R^{12}N(CH_2)_m$,

$$R^{11}R^{12}N\overset{O}{\overset{\|}{C}}(CH_2)_m \quad ou \quad R^{11}R^{12}N\overset{O}{\overset{\|}{C}}O(CH_2)_m$$

dans lesquels $R^{11}$ et $R^{12}$ représentent chacun indépendamment un atome d'hydrogène, un radical alkyle en $C_{1-3}$, hydroxyalkyle en $C_{2-3}$, ou ensemble avec l'atome d'azote auquel ils sont fixés, ils forment un hétérocycle choisi parmi pipéridinyle, pyrrolidinyle, pipérazinyle, morpholinyle ou thiomorpholinyle;
(5) $R^{13}S(0)_n(CH_2)_m$ dans lequel $R^{13}$ est un atome d'hydrogène, un radical alkyle en $C_{1-3}$, amino, alkylamino en $C_{1-3}$, ou di(alkyl en $C_{1-3}$)amino ; et a, b et c représentent chacun des liaisons simples ou l'un d'eux représente une liaison double ou a et c représentent une liaison double ;
ou son sel pharmaceutiquement acceptable

2. Composé selon la revendication 1, dans lequel $R^1$ et $R^5$ sont indépendamment choisis parmi :
(1) alkyle en $C_{1-10}$ ;
(2) alkyle substitué en $C_{1-10}$ dans lequel un (ou plusieurs) substituant est choisi parmi :
(a) halogène,
(b) hydroxy,
(c) alcoxy en $C_{1-10}$,
(d) alcoxycarbonyle en $C_{1-5}$,
(e) acyloxy en $C_{1-5}$,

(f) cycloalkyle en $C_{3-8}$,
(g) phényle,
(h) phényle substitué dont les substituants sont X et Y ; et
(i) oxo ;
(3) cycloalkyle en $C_{3-8}$ ;
(4) cycloalkyle en $C_{3-8}$ substitué dans lequel un substituant est choisi parmi :
(a) alkyle en $C_{1-10}$,
(b) alkyle substitué en $C_{1-10}$ dans lequel le substituant est choisi parmi :
(i) halogène,
(ii) hydroxy,
(iii) alcoxy en $C_{1-10}$,
(iv) acyloxy en $C_{1-5}$,
(v) alcoxycarbonyle en $C_{1-5}$,
(vi) phényle,
(vii) phényle substitué dans lequel les substituants sont X et Y ; et
(viii) oxo ;
(c) halogène,
(d) hydroxy,
(e) alcoxy en $C_{1-10}$,
(f) alcoxycarbonyle en $C_{1-5}$,
(g) acyloxy en $C_{1-5}$,
(h) phényle,
(i) phényle substitué dont les substituants sont X et Y ;
(5) phénylamino ;
(6) phénylamino substitué dont les substituants sont X et Y ;
(7) phénylalkylamino en $C_{1-10}$ ; et
(8) phénylalkylamino en $C_{1-10}$ substitué dont les substituants sont X et Y.

3. Composé selon la revendication 2, dans lequel $R^1$ et $R^5$ sont indépendamment choisis parmi :
(1) alkyle en $C_{1-10}$ ;
(2) cycloalkyle en $C_{3-8}$ ;
(3) phénylamino ; et
(4) phénylamino substitué dont les substituants sont X et Y ;
et $R^3$ et $R^4$ sont indépendamment choisis parmi :
(1) alkyle en $C_{1-10}$ ;
(2) cycloalkyle en $C_{3-8}$ ; et
(3) phényle.

4. Composé selon la revendication 3, dans lequel a et c représentent des doubles liaisons.

5. Composé selon la revendication 4, qui est choisi parmi :
(1) 6(R)-[8-[2(S)-(2,2-diméthylbutyryloxy)-2(S)-méthyl-6(S)-(1-hydroxyéthyl)-1,2,6,7,8,8a (R)-hexahydro_naphtyl-1(S)]éthyl]-4(R)-hydroxy-3,4,5,6-tetrahydro-2H-pyran-one;
(2) 6(R)-[2-(8(S)-(2,2-diméthylbutyryloxy)-2(S)-méthyl-6(R)-(1-hydroxyéthyl)-1,2,6,7,8,8a-(R)-hexahydro_naphtyl-1(S)]éthyl]-4(R)-hydroxy-3,4,5,6-tetrahydro-2H-pyran-2-one;
(3) 6(R)-[2-[8(S)-(2-méthylbutyryloxy)-2(S)-méthyl-6(S)-(1-hydroxyéthyl)-1,2,6,7,8,8a(R)-hexahydro_naphtyl-1(S)]ethyl]-4(R)-hydroxy-3,4,5,6-tetrahydro-2H-pyran-2-one;
(4) 6(R)-[2-[8(S)-(2-méthylbutyryloxy)2(S)-méthyl-6(R)-(1-hydroxyéthyl)-1,2,6,7,8,8a(R)-tetrahydro_naphtyl-1(S)]éthyl]-4(R)-hydroxy-3,4,5,6-tetrahydro-2H-pyran-2-one;
et les acides dihydroxyliques correspondants à noyaux ouverts et leurs esters.

6. Composé selon la revendication 3, dans lequel a, b, et c sont des liaisons simples ou bien l'un des symboles a, b ou c représente une liaison double.

7. Composé selon la revendication 6, qui est choisi parmi :
(1) 6(R)-[2-[8(S)-(2,2-diméthylbutyryloxy)2(S)-méthyl-6(S)-(1-hydroxyéthyl)-1,2,3,4,4a(S),5,6,7,8,8a(S)-decahydro-naphtyl-1(S)]éthyl]-4(R)-hydroxy-3,4,5,6-tetrahydro-2H-pyran-2-one;
(2) 6(R)-[2-[8(S)-(2,2-diméthylbutyryloxy)2(S)-méthyl-6(R)-(1-hydroxyéthyl)-1,2,3,4,4a(S),5,6,7, 8,8a(S)-decahydro-naphtyl-1(S)]éthyl]-4(R)-hydroxy-3,4,5,6-tetrahydro-2H-pyran-2-one;
(3) 6(R)-[2-[8(S)-(2,2-diméthylbutyryloxy)2(S)-méthyl-6(S)-(1-hydroxyphénylméthyl)-1,2,3,4, 4a(S),5,6,7, 8,8a(S)-decahydro naphtyl-1(S)]éthyl]-4(R)-hydroxy-3,4,5,6-tetrahydro-2H-pyran-2-one;

(4) 6(R)-[2-[8(S)-(2,2-diméthylbutyryloxy)-2(S)-méthyl-6(S)-(1-oxoéthyl)-1,2,3,4,4a(S),5,6,7,8,8a(S)-decahydro naphtyl-1(S)]éthyl]-4(R)-hydroxy-3,4,5,6-tetrahydro-2H-pyran-2-one;

(5) 6(R)-[2-(8(S)-(2,2-diméthylbutyryloxy)-2(S)méthyl-6(R)-(1-hydroxyéthyl)-1,2,3,4,6,7,8,8a(R)-octahydro naphtyl-1(S)]éthyl]-4(R)-hydroxy-3,4,5,6-tetrahydro-2H-pyran-2-one;

et les acides dihydroxyliques correspondants à noyaux ouverts ainsi que leurs esters.

8. Composition pharmaceutique hypocholestérolémique, hypolipidémique comprenant un véhicule pharmaceutiquement acceptable et une quantité efficace non toxique d'un composé tel que défini dans la revendication 1.

9. procédé de préparation d'un composé répondant à la formule structurale générale I ci-après selon la revendication 1, qui consiste :

(A) à traiter un composé de formule structurale:

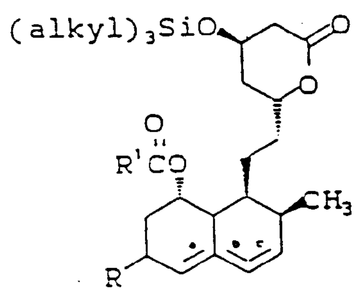

avec soit le fluorure de n-tetrabutylammonium dans l'acide acétique et un solvant inerte soit l'acide fluorhydrique aqueux au sein d'un solvant inerte ; et

(B) à former facultativement le composé de formule structurale générale II ou un sel pharmaceutiquement acceptable de celui-ci en traitant le composé de formule I avec :

(1) un hydroxyde de métal alcalin dans un solvant aqueux pour préparer un sel de métal alcalin ; ou

(2) un hydroxyde de métal alcalin dans un solvant aqueux pour préparer le sel de métal alcalin et à acidifier ce sel pour former l'acide dihydroxylique libre ; ou

(3) un hydroxyde de métal alcalin dans un solvant aqueux pour préparer le sel de métal alcalin et à estérifier ce sel avec un alcanol en $C_{1-5}$ pour former l'ester alkylique en $C_{1-5}$.